# EUROPEAN PATENT APPLICATION

(11) **EP 2 371 365 A1**
(43) Date of publication of application: **05.10.2011**
(21) Application number: 10178620.0
(22) Date of filing: 20.12.2005
(51) Int. Cl.: A61K 31/429, A61K 47/40, A61K 9/00, A61P 35/00, A61P 43/00, A61K 9/19, A61K 47/48, B82Y 5/00

(54) **Compositions comprising an epothilone and production methods**

(30) Priority: 23.12.2004 EP 04090516
(62) Divisional of application: 05823451.9
(71) Applicant: Bayer Pharma Aktiengesellschaft, 13353 Berlin (DE)
(72) Inventor: Uffrecht, Anka, 13465, Berlin (DE); Sachse, Andreas, 13465, Berlin (DE); Reer, Olaf, 13465, Berlin (DE); Thomsen, Jens, 10781, Berlin (DE); Renz, Mathias, 13465, Berlin (DE); Sprenger, Claudia, 12059, Berlin (DE)

(57) **Abstract**

The present invention concerns methods for the production of pharmaceutical formulations of Epothilones suitable for being administered parenterally, such as intravenously.

## Description

### FIELD OF INVENTION

The present invention concerns methods for producing pharmaceutical formulations of Epothilones and compositions suitable for being administered parenterally, such as intravenously.

### BACKGROUND OF THE INVENTION

Epothilones belong to a natural class of substances with cytotoxic effect and which may be administered parenterally. Unfortunately, the natural substances are not sufficiently stable, either chemically or metabolically, for being developed as pharmaceutical agents. The lactone structure in the Epothilone backbone makes the molecule susceptible to degradation, especially at higher pH values such as above neutral pH. Furthermore, the Epothilones are highly lipophilic substances and practically insoluble in water, which render this class of compounds especially difficult to administer intravenously. Parenterally administered formulations require the Epothilones being completely dissolved in a solvent compatible with physiological fluids, such as blood.

Several efforts have been made in order to make available new Epothilone derivatives, which are both chemically and metabolically stable enough for the development of pharmaceutical agents. Furthermore, the Epothilone derivatives should be superior to natural derivatives in terms of their therapeutic range, their selectivity of action and/or undesirable toxic side effects and/or their active strength. WO 00/66589 describes such superior Epothilones, wherein the carbon atom 6 of the Epothilone depicted on page 1 of WO 00/66589 is provided with an alkenyl, alkinyl or an epoxy radical. However, such additional side-chains make such Epothilones even more lipophilic and poorly wettable In solvents rendering the formulation of pharmaceuticals as an even greater challenge to the skilled person in the pharmaceutical art.

Several methods for Increasing the solubility of sparingly water-soluble drugs so as to produce parenteral compatible formulations have been described. US 6,407,079 (Janssen Pharma) describes injectable formulations, wherein a partially etherified β-cyclodextrin (hydroxyethyl, hydroxypropyl, dihydroxypropyl, methyl or ethyl ethers) is added to a drug that is instable or only sparingly soluble in water. The resulting inclusion compound is more water soluble than the drug itself.

Sulfoalkyl ether cyclodextrins and derivatives thereof for use as solubilising agents for water insoluble drugs are previously described in US 5,376,645 and US 5,134,127.

WO 99/396945 describes Epothilones of type A and B formulated as an infusion concentrate or as a lyophilised composition. Prior to administration, the Epothilone should be dissolved in particular solvents, such as PEG/water mixtures, propyleneglycol/water or ethanol/water. The solubility of the Epothilone increases significantly by applying such solvent mixtures. β-Cyclodextrin or mannitol is added as a bulking excipient.

WO 2004/032866 describes lyophilised compositions comprising Epothilones, preferably Epothilone D together with β-cyclodextrins, including sulfoalkyl ether cyclodextrins.

The production of epothilones, and derivatives is carried out according to the methods that are known to one skilled in the art, as they are described in, for example, DE 19907588, WO 98/25929, WO 99/58534, WO 99/2514, WO 99/67252, WO 99/67253, WO 99/7692, EP 99/4915, WO 00/1333, WO 00/66589, WO 00/49019, WO 00/49020, WO 00/49021, WO 00/71521, WO 00/37473, WO 00/57874, WO 01/92255, WO 01/81342, WO 01/73103, WO 01/64650, WO 01/70716, US 6204388, US 6387927, US 6380394, US 02/52028, US 02/58286, US 02/62030, WO 02/32844, WO 02/30356, WO 02/32844, WO 02/14323, and WO 02/8440. Particularly interesting epothilones of the present invention may be produced according to WO 00/66589.

It is an objective of the present invention to provide a composition comprising an Epothilone derivative and which is intended for being administered parenterally, preferably Intravenously. The composition should be stable, at least with respect to the Epothilone, during storage and reconstituted, if necessary, by adding a solvent compatible with blood and suitable for parenteral administration without the need of adding surfactants and organic solvents.

### SUMMARY OF THE INVENTION

The present invention relates to parenteral dosage forms comprising Epothilones and the manufacture thereof in which the final dosage form or the manufacturing process meets the following characteristics:
- Fast solving of the Epothilone in a liquid, which can be removed during lyophilization conditions, so as to limit the loss of intact Epothilone during the solving process.
- Lyophilising of the liquid Epothilone composition without loosing intact Epothilone
- Lyophilisate is sufficiently hard.
- Complete and fast dissolution of the lyophilised cake in a physiologically acceptable solvent so as to form a re-constituted composition ready to be administered or further diluted with a suitable physiologically acceptable solvent.
- Chemically stable lyophilised compositions, at least with respect to the stability of Epothilone.
- Physically stable lyophilised compositions.
- Chemically stable re-constituted solution, at least with respect to the stability of Epothilone.
- Physically stable re-constituted solution.
- High concentration of Epothilone in lyophilised cake and/or re-constituted solution
- High Maximum Tolerated Dose following parenteral administration.

By the present invention lyophilised compositions as well as reconstituted compositions thereof are provided, which as an overall concept comprise an Epothilone or a derivative thereof or mixtures thereof in combination with a cyclodextrin.

Unlike previously described parenterally adminsterable compositions of Epothilones, the compositions of the present invention allow for compositions resulting from reconstitution of the lyophilisate in simple solvents like water or saline water and optionally further diluting with a glucose or dextrose solution. Addition of an organic solvent and a glycol is not required in order to get the Epothilone easily dissolved. As mentioned in WO 2004/032866, the reconstitution of lyophilisates requires mixtures of water, ethanol and a glycol. Lyophilisates of WO 99/39694 also require an organic solvent. Obviously, the lyophilisates of the present invention is advantageous to those previously described in that the preparation of the final reconstituted composition only requires water for injection or saline.

In one aspect the invention relates to compositions comprising an Epothilone or mixtures of Epothilones, wherein the Epothilone is provided with a more lipophilic side chain than in the naturally occurring Epothilones. According to the formula I depicted herein, the lipophilic side chain refers to R⁴ and is located at the carbon atom numbered 10 given that the Epothilone is fused with an epoxy ring or located at the carbon atom numbered 7 given that R⁷ and R⁸ are a hydrogen atom or taken together is an additional bond. In WO 00/66589 this carbon atom was numbered 6. The compositions of the invention further comprise a cyclodextrin, preferably a β-cyclodextrin derivative that is etherified with hydroxyalkyl groups and/or sulfoalkyl groups resulting in hydroxyalkylated cyclodextrins or sulfoalkylated cyclodextrins.

It has been found that a general concept of formulating Epothilones suitable for being parenterally administered is enabled by the proper selection of excipients providing the required tonicity, pH, stabilisation of the composition comprising the cyclodextrin and the Epothilone, chemically and physically stability during lyophilisation, chemically and physically stability of the lyophilised composition. This concept Is particularly suitable for Epothilones with very poor wettability properties and/or low water solubility, such as the particular derivatives described by formula I herein. Typical examples on further excipients are a tonicifying agent, a filler, a cryoprotectant, a lyoprotectant and a pH regulating agent. In preferred embodiments of the invention, the further pharmaceutically acceptable excipient is selected from mannitol; sorbitol; xylitol; 2-Amino-2-hydroxymethyl-1,3-propandiol; the acid form or salts of citric acid, acetic acid, histidine, malic acid, phosphoric acid, tartaric acid, succinic acid, MES, HEPES, imidazole, lactic acid, glutaric acid and/or glycylglycine, preferably mannitol and/or trometamol (TRIS).

The inventors have also found that especially useful cyclodextrins apply to sulfoalkyl ether cyclodextrins in that such resulting compositions possess the desired tonicity without the need of adding further fillers or tonicity modifying agents.

Therefore, in another aspect the invention relates to compositions comprising an Epothilone and a sulfoalkylated cyclodextrin (e. g., a sulfopropylated and a sulfobutylated cyclodextrin).

A still further aspect of the invention relates to a composition comprising an Epothilone according to formula I described herein and a β-cyclodextrin. The cyclodextrin Is preferably a hydroxyalkylated β-cyclodextrin or a sulfoalkylated β-cyctodextrin as mentioned above.

Another preferred aspect of the invention relates to a composition comprising an Epothilone according to formula I described herein and a sulfoalkylated β-cyclodextrin, preferably a sulfobutylether-β-cyclodextrin.

A preferred aspect of the invention relates to a composition comprising an Epothilone according to formula I described herein and a hydroxylated-, most preferred a partly etherified Hydroxypropyl-β-Cydodextrin.

Furthermore, the inventors have provided a method of producing the lyophilised composition of the invention, which overcome the initial critical step of solving the very hydrophobic Epothilones, namely the initial wetting of the Epothilone in a suitable liquid before the solubilising process can take place afterwards.

This process enables fast and complete solving of the Epothilone and further allows for the Epothilone to stay stable in solution, in a sufficient period of time, before the removal of solvent takes place.

By example, the process of solving the Epothilone is much faster than the one described in WO 99/39694, Example 10. In fact the use of the process as described in Example 10 of WO 99/39694 would not work for the Epothilones defined herein. It would take days to get the Epothilones as described herein into solution, during which time hydrolysis would take place. Considerable loss of the Epothilone is therefore resulting and the described process is not suitable for industrial production.

Accordingly, a still another aspect of the invention relates to the manufacturing of a solid composition, such as a lyophilisate, which may be formed from solutions (hereinafter referred to as "original solutions") comprising an Epothilone as described herein, a cyclodextrin as described herein and optionally at least one further pharmaceutically acceptable excipient as defined herein.

The specific characteristic of the method provided is that the Epothilone may be solved In the solvent suitable for the lyophilisation process during a shorter period of time than known from the state of the art, short enough to make sure that the Epothilone remains stable and no decomposition takes place.

The period of time is envisaged to be 0.5 to 5 hours, preferably 0,5 to 3 hours, more preferably 1 to 2,5 hours.

In one embodiment, the method for producing a composition of the invention (process) comprises the steps of
a) solving an Epothilone - no matter whether crystalline or amorphous - as defined herein in an organic solvent, such as an alcohol (preferably ethanol); and
b) solving a cyclodextrin as defined herein in aqueous solution, optionally together with at least one further pharmaceutically acceptable ingredient as defined herein, such as mannitol and /or tromethamol; optionally
c) adjusting the pH of the resulting mixture of b) to a pH ranging between 5 and 9, preferably 6 and 8, such as about 7.4 using an inorganic acid, such as hydrochloric acid; and
d) mixing the resulting solutions a) and b) or a) and c); and optionally
e) carrying out sterile filtering of d) to achieve the so-called "original solution"
f) removing the solvents from the "original solution" to provide a solid composition.
   In another embodiment, the method for producing a composition of the invention (process) comprises the steps of
   a) solving an Epothilone - no matter whether crystalline or amorphous - as defined herein in an organic solvent, such as an alcohol (preferably ethanol); and
   b) evaporating said organic solvent; and
   c) solving a cyclodextrin as defined herein in aqueous solution, optionally together with at least one further pharmaceutically acceptable ingredient as defined herein, such as mannitol and /or tromethamol; optionally
   d) adjusting the pH of the resulting mixture of b)to a pH ranging between 5 and 9, preferably 6 and 8, such as about 7.4 using an inorganic acid, such as hydrochloric acid; and
   e) solving the resulting powder b) in the resulting solution c) or d); and optionally
   f) carrying out sterile filtering of e) to achieve the so-called "original solution"
   g) removing the solvent from the "original solution" to provide a solid composition.

The organic solvents suitable for step a) if the second step b) is evaporation of the solvent can be selected from polar aprotic or protic solvents for example can be selected from halogenated hydrocarbons such as monochloromethane, dichloromethane; alcohols such as methanol, ethanol, propanol; acetone. Preferred solvents are methylenechloride and ethanol.

Removal of a solvent in the sense of the invention includes all techniques for removal of a solvent or solvents known to one skilled in the art also such as freeze drying, lyophilization, vacuum drying or evaporation but is not limited thereto.

A preferred aspect of the invention relates to the methods for producing a composition comprising an epothilone (processes) mentioned above wherein the Epothilone used is in an amorphous form and the composition obtainable thereof.

Amorphous is a solid phase without crystal lattice. It can be proved by X-ray powder diffraction.

Epothilone can be obtained in amorphous form for example by solving crystalline epothilone in an organic solvent and subsequently removing the solvent thereof.

A preferred aspect of the invention relates to the methods for producing a composition comprising an epothilone (processes) mentioned above wherein the Epothilone used is in an amorphous form or it is transferred into an amorphous form and the cyclodextrin is hydroxypropyl-β-cyclodextrin and the composition obtainable thereof.

A preferred aspect of the invention relates to the methods for producing a composition comprising an epothilone (processes) mentioned above wherein the Epothilone used is in an amorphous form or it is transferred into an amorphous form and the cyclodextrin is sulfobutylether-β-cyclodextrin and the composition obtainable thereof.

A preferred aspect of the invention relates to the methods for producing a composition comprising an epothilone (processes) mentioned above wherein the Epothilone used is the Epothilone derivative (1S, 3S, 7S,10R, 11S, 12S, 16R)-7,11-dihydroxy-3-(2-methyl-benzothiazol-5-yl)-10-(prop-2-en-1-yl)-8,8,12,16-tetramethyl-4,17-dioxabicyclo[14.1.0]heptadecane-5,9-dione, in addition the epothilone is in an amorphous form or is transferred into an amorphous form and the cyclodextrin is hydroxypropyl-β-cyclodextrin and the composition obtainable thereof.

A preferred aspect of the invention relates to the methods for producing a composition comprising an epothilone (processes) mentioned above wherein the Epothilone used is the Epothilone derivative (1S, 3S, 7S,10R, 11S, 12S, 16R)-7,11-dihydroxy-3-(2-methyl-benzothiazol-5-yl)-10-( prop-2-en-1-yl)-8,8,12,16-tetramethyl-4,17-dioxabicydo[14.1.0]heptadecane-5,9-dione, in addition the epothilone is in an amorphous form or is transferred into an amorphous form and the cyclodextrin is sulfobutylether-β-cyclodextrin and the composition obtainable thereof.

Another preferred aspect of the invention relates to the compositions obtainable by the methods for producing a composition comprising an epothilone (processes) mentioned above.

Still another aspect the invention relates to compositions containing Epothilone and hydroxylalkylated cyclodextrin in a specific molar ratio of 1:8 to 1:100, preferred 1:11 to 1:80 which is equivalent to the mass ratio of 1:21 to 1:300, preferred 1:29 to 1:200. Especially preferred are the ratios obtained by the examples herein.

Still another aspect the invention relates to compositions containing Epothilone and sulfobutylether-cyclodextrin in a specific molar ratio of 1:9 to 1:100, preferred 1:9 to 1:50 which is equivalent to the mass ratio of 1:38 to 1:300, preferred 1:38 to 1:200. Especially preferred are the ratios obtained by the examples herein.

### DETAILED DESCRIPTION OF THE INVENTION

Compositions provided by the present invention are compositions with sufficient and high solubility of an Epothilone by combining an Epothilone, preferably used in an amorphous form, and a cyclodextrin in combination with further adapted excipients and/or by selecting the cyclodextrin carefully. The compositions are preferably further formulated in a form allowing parenteral administration of the composition, such as in the form of a solid solution, such as lyophilized composition, that is further transformed into a composition resulting from reconstitution of the lyophilisate with a suitable solvent.

The term "formulated In a form for parenteral use" generally refers to Injectable compositions. Injectable compositions can be administered intravenously, subcutaneosly or by infusion. In preferred embodiments of the invention, the injectable compositions are for intravenous administration.

The terms "lyophilised composition", "lyophilised cake" and "lyophilisate" are interchangeable terms and are used herein to mean the solid composition resulting from processing a liquid composition, such as a solution comprising the Epothilone solved or at least partly solved, under conditions including at least one step of freezing the solution, followed by sublimation of the solvent(s) in a vacuum (main drying) and optionally and additionally evaporation/removal of the solvent(s) during secondary drying and optionally postdrying e.g. under the following conditions:
- Freezing to -45°C at 1013 mmbar for up to 24 hours, preferably for 5 hours,
- first main drying phase to 15°C at 8.9X10⁻² mmbar for 60 hours, preferably for 48 hours,
- second main drying phase at 25°C at 8.9x10⁻² mmbar for 2 hours, preferably for 1 hour and
- postdrying phase at 25°C at 6.5 x10⁻³ mmbar for 10 hours, preferably for 6 hours using for example a freeze dryer Fa. Hof, type COM 0590.

The terms "lyophilisation" and "freeze-drying" are denoted to mean a process upon where liquid is removed from a dissolved or at least partly dissolved composition under conditions involving at least one step of freezing the solution, followed by sublimation of the solvent(s) in a vacuum (main drying) and optionally additionally evaporation of the solvent(s) during secondary drying, and optionally postdrying for example under the conditions mentioned in the paragraph above.

The term "reconstituted solution" refers to a liquid composition resulting from completely dissolving a lyophilisate, which can be a solid solution, in a solvent such as water (water for injection), saline or sterile Ringer's solution. The solvent may include further excipients so as to make the reconstituted composition compatible with physiologically relevant fluids, such as blood. The reconstituted compositions are intended for being ready to be administered parenterally or to be further diluted before use.

By the term "sulfoalkyl ether cyclodextrin" is intended a derivative obtained by introducing an anionic-type substituent, such as a (C₂-₆ alkylene)-SO₃ anionic substituent onto the cyclodextrin. The sulfoalkyl derivative of cyclodextrin can be a single derivative or a mixture of derivatives. As the cyclodextrin derivatives are functionalised with (C₂₋₆ alkylene)-S03 groups, the derivatives are charged species. The sulfoalkyl ether cyclodextrin are either substituted at least at one of the primary hydroxyl groups or they are substituted at both the primary hydroxyl groups and at the 3-positioned hydroxyl group. Substitution at the 2-position is theoretically possible.

The term hydroxyalkylated β-cyclodextrin, especially hydroxypropyl-β-cyclodextrin means a cyclodextrin derivative wherein under defined conditions (publicly disclosed by the supplier, e.g. Roquette GmbH, of the cyclodextrin) the free hydroxyl groups of the β-cyclodextrin are partly or completely etherified resulting from controlled reaction of alkylenoxide, especially propylenoxide, and native beta-cyclodextrin under base catalysis forming hydroxymethyl, hydroxyethyl, or hydroxypropyl groups respectively. In the event of partly etherified hydroxyl groups the resulting β-cyclodextrin is characterized by its average molar degree of substitution (MS) which is the average number of moles of hydroxypropyl groups per anhydroglucopyranose unit. This is due to the manufactures information not to be mixed up with the degree of substitution (DS) or the total degree of substitution (TDS) which represents the average number of substituted hydroxyls per anhydroglucopyranose unit.

Typically the solvent for reconstitution is an aqueous solution comprising 75-100% of water by volume, preferably 85%-100% by volume, more preferably 90-100% by volume, most preferably 95-100% by volume. The solvent may comprise further excipients such as inorganic salts like sodium chloride so as to be compatible with physiologically relevant fluids.

The term "solubility" refers to the solubility of Epothilone in a solvent.

According to the invention, an Epothilone needs to be combined with one or more agents that increase the solubility of the Epothilone in water. As a first solubilising agent, a cyclodextrin has been found to improve the solubility of an Epothilone. However, in some cases further excipients may be added to further Increase the solubility of the Epothilone or to limit degradation of Epothilone during preparation of the parenteral composition. For example further excipients may be added in order to stabilise the Epothilone during admixing of excipients, during removing of liquid, or during storage or after reconstitution.

Therefore, in one aspect the invention provides a composition comprising an Epothilone, a cyclodextrin and at least one pharmaceutically acceptable excipient selected from a tonicifying agent, a filler, a cryoprotectant, a lyoprotectant and a pH regulator.

In another aspect the Invention provides a composition comprising an Epothilone, such as an Epothilone derivative as defined by formula I, and a cyclodextrin, the Epothilone Is of formula I, 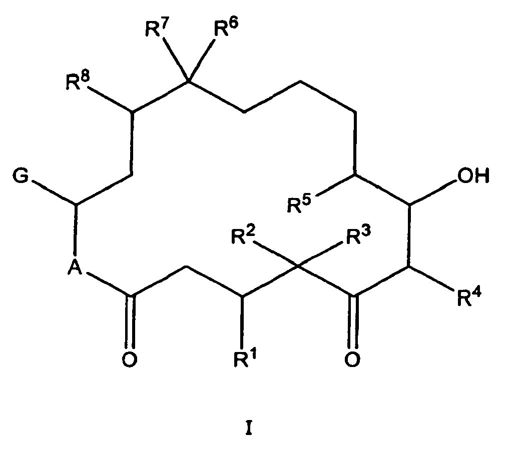 wherein
- R¹: means hydrogen, OR^{1a}, or Halogen, where R^{1a} is hydrogen, SO₂-alkyl, S0₂-aryl, or SO₂-aralkyl,
- R², R³: are Independently C₁-C₁₀ alkyl,
- R⁴: means -(CH₂)ᵣ-C=C-(CH₂)ₚ-R^{4a}, -(CH₂)ᵣ-CH=CH-(CH₂)ₚ-R^{4a},

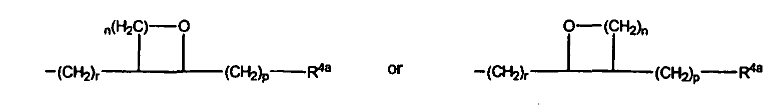

- n: means 0 to 5,
- r: is 0 to 4,
- p: is 0 to 3,
- R^{4a}: means hydrogen, C₁-C₁₀ alkyl, C₆-Cₙ aryl or C₇-C₂₀ aralkyl, each optionally substituted;C₁-C₁₀ acyl, or, if p > 0, additionally a group OR^{4b}, R^{4b} means hydrogen or a protective group PG;
- R⁵: means C₁-C₁₀ alkyl,
- R⁶: means hydrogen or optionally substituted C₁-C₁₀ alkyl,
- R⁷, R⁸: each mean a hydrogen atom, or taken together an additional bond or taken together an oxygen atom,
- G: means a group X=CR⁹- or a bi- or tricyclic aromatic heterocyclic radical,
- R⁹: means hydrogen, halogen, CN, or a C₁-C₂₀ alkyl,
- X: means a grouping CR¹⁰R¹¹, whereby R¹⁰, R¹¹ are the same or different and stand for hydrogen, a C₁-C₂₀ alkyl, C₆-C₁₂ aryl, or C₇₋₂₀ aralkyl radical each optionally substituted; or R¹⁰ and R¹¹ together with the methylene carbon atom jointly stand for a 5- to 7-membered carbocyclic ring;
- A: means a group -O- or -NR¹²-,
- R¹²: means hydrogen or C₁-C₁₀ alkyl.

The Epothilone according to formula I may be fused with an epoxide ring In the event where R⁷, R³ taken together are an oxygen atom or may exist as one ring system in the event where R⁷, R⁸ each mean a hydrogen atom or taken together mean an additional bond.

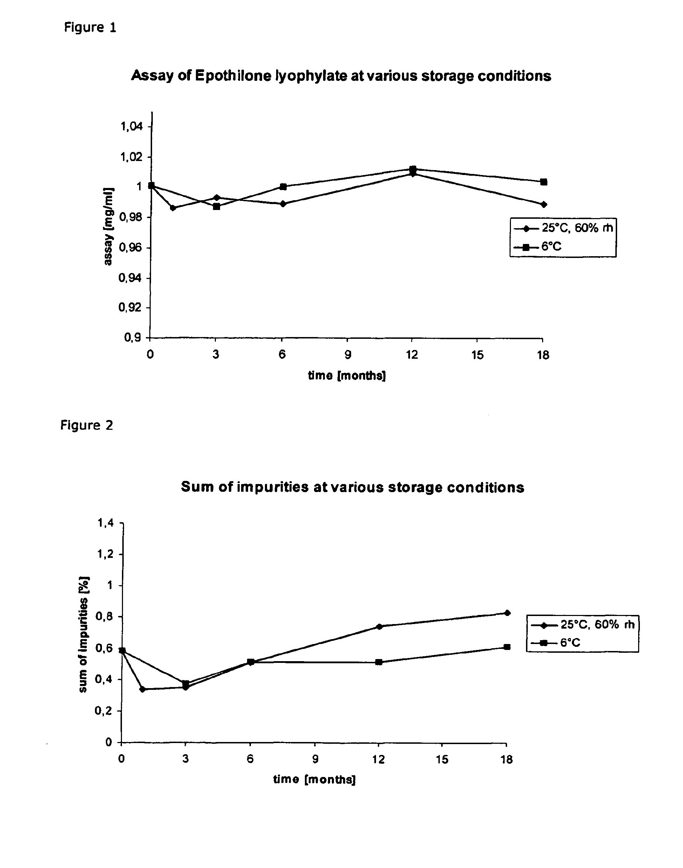

The numbering of the carbon atoms in the Epothilone skeleton will depend on whether the Epothilone is fused with an additional ring, such as an epoxide ring. The formula II as depicted below shows the numbering of carbon atoms In both situations. Figures 1 to 16 refer to the numbering of an Epothilone skeleton, wherein R⁷, R⁸ taken together make an epoxide ring. Figures (1) to (16) refer to the numbering of an Epothilone skeleton, wherein R⁷, R⁸ each mean a hydrogen atom or taken together mean an additional bond.

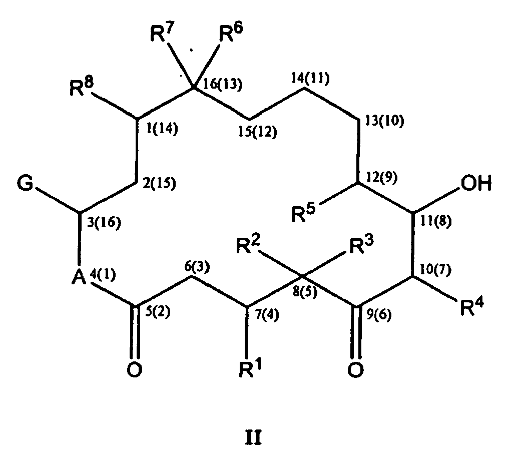

The term "halogen" Includes fluorine, chlorine, bromine and iodine.

The term SO₂-alkyl is intended to mean "C₁-C₁₀ alkyl" monosubstituted with -SO₂ group.

The term SO₂-aryl is intended to mean "C₆-C₁₂ aryl" monosubstituted with -SO₂ group.

The term SO₂-aralkyl is intended to mean "C₆-C₁₂ aryl" substituted with one -SO₂ group and with one or two "C₁-C₁₀ alkyl".

The term "C₁-C₁₀ alkyl" is intended to mean a linear or branched saturated hydrocarbon chain wherein the longest chains have from one to ten carbon atoms, such as methyl, ethyl, n-propyl, isopropyl, n-butyl, isobutyl, sec-butyl, tert-butyl, pentyl, isopentyl, neopentyl, hexyl, heptyl, octyl, undecacyl, dodecyl, etc. The C₁-C₁₀ alkyl chain of the present invention may be optionally substituted.

Likewise, the term "C₁-C₂₀ alkyl" is intended to mean a linear or branched saturated hydrocarbon chain wherein the longest chains have from one to twenty carbon atoms.

The term "C₆-C₁₂ aryl "is intended to mean a substituted or unsubstituted carbocyclic aromatic radical or heterocyclic radical consisting of between 6 and 12 carbon atoms. Moreover, the term "aryl" includes fused ring systems wherein at least two aryl rings share at least one chemical bond. Illustrative examples of "aryl" rings include phenyl, naphthalenyl. A preferred aryl group is phenyl and substituted phenyl groups, carrying one or two, same or different, of the substituents listed above. The preferred pattern of substitution is *para* and/or *meta.* Representative examples of aryl groups include, but are not limited to, phenyl, 3-halophenyl, 4-halophenyl, 3-hydroxyphenyl, 4-hydroxyphenyl, 3-aminophenyl, 4-aminophenyl, 3-methylphenyl, 4-methylphenyl, 3-methoxyphenyl, 4-methoxyphenyl, 3-cyanophenyl, 4-cyanophenyl, dimethylphenyl, naphthyl, hydroxynaphthyl, hydroxymethylphenyl, trifluoromethylphenyl, alkoxyphenyl.

The term "aromatic heterocyclic radical" Is intended to mean a substituted or unsubstituted aromatic heterocyclic radical consisting of between 6-12 carbon atoms containing one or more heteroatoms. Representative examples of aromatic heterocyclic radicals are furyl, thienyl, pyridyl, pyrazolyl, pyrimidinyl, oxazolyl, pyridazinyl, pyrazinyl, quinolyl, thiazolyl, benzothiazolyl, benzoxazolyl, quinoline which can be substituted in one or more places by halogen, OH, O-alkyl, CO₂H, CO₂-alkyl, NO₂, N₃, CN, C₁-C₁₀ alkyl, C₁-C₁₀ acyl, C₁-C₁₀ acyloxy groups. Heteroatoms in the heteroaryl radicals can be oxidized; thus, for example, the benzothiazole ring can be present In the form of N-oxide. Preferred aromatic heterocyclic radicals include benzothiazolyl, benzoxazol, and quinoline; more preferably C₁-C₁₀ alkyl substituted benzothiazolyl.

The term "C₇-C₂₀ aralkyl" is intended to mean a carbocyclic aromatic ring or ring system consisting of between 6 and 12 carbon atoms, preferably 6 to 10, and in the alkyl chain 1 to 8, preferably 1 to 4 atoms, wherein at least one aryl ring share at least one chemical bond with a C₁-C₈ alkyl. As aralkyl radicals, for example, benzyl, phenylethyl, naphthylmethyl, naphthylethyl, furylmethyl, thienylethyl, and pyridylpropyl are suitable. The rings can be substituted in one or more places by halogen, OH, O-alkyl, CO₂H, CO₂-alkyl, NO₂, N₃, CN, C₁-C₁₀ alkyl, C₁-C₁₀ acyl, C₁-C₁₀ acyloxy groups.

The term "C₁-C₁₀ acyl" is intended to mean a linear or branched saturated hydrocarbon chain wherein the longest chains have from one to ten carbon atoms and wherein one of the carbon atom is a C(=O)O radical.

The alkoxy groups that are contained in X in general formula I are in each case to contain 1 to 20 carbon atoms, whereby methoxy, ethoxy, propoxy, isopropoxy and t-butyloxy groups are preferred.

The term "Epothilone" and "Epothilone*" in general is meant to encompass all kinds of substances belonging to the class of Epothilones, naturally or synthetically made, either as a single Epothilone or a mixture of Epothilones. That is to say that the term "Epothilone" refers to any Epothilone, such as Epothilone A, Epothilone B, Epothilone C, Epothilone D, Epothilone E, Epothilone F, analogs, derivatives, salts and mixtures thereof. Preferably, the Epothilone is Epothilone A, Epothilone B, Epothilone C, Epothilone D or derivatives thereof or salts thereof or mixtures thereof. In some particular embodiments, the Epothilone is an Epothilone B derivative according to formula I above.

In some interesting embodiments of the invention, the Epothilone is an Epothilone derivative, wherein the carbon atom 10(7) in the 16-membered macrolide system is provided with an alkenyl, alkinyl or epoxide group as defined above (R⁴ in formula I) instead of the methyl group in the natural occuring Epothllones. Thus, an Epothilone of the present invention refers in general to a derivative of any Epothilone, such as Epothilones A, B, C, D, E or F, in which carbon atom 10(7) of the 16-membered macrolide system is provided with an alkenyl, alkinyl or epoxide group as defined by R⁴ in formula I. As said Epothilones B are of particular interest to the present invention, which means that the above-mentioned derivatives are preferably derivatives of Epothilone B.

In still interesting embodiments of the invention, the Epothilone is an Epothilone derivative selected from:
(1S, 3S (E), 7S, 10R, 11S, 12S, 16R)-7,11-dihydroxy-10-(prop-2-yn-1-yl)-3-(1-methyl-2-(2-methylthiazol-4-yl)ethenyl)-8,8,12,16-tetramethyl-4,17-dioxabicyclo [14.1.0] heptadecane-5,9-dione;
(1S, 3S, 7S, 10R, 11S, 12S, 16R)-7,11-dihydroxy-10-(prop-2-en-1-yl)-3-(2-methyl-benzoxazol-5-yl)-8,8,12,16-tetramethyl-4,17-dioxabicyclo [14.1.0] heptadecane-5,9-dione;
(1S, 3S (E), 7S, 10R, 11R, 12S, 16R)-7,11-dihydroxy-10-(prop-2-yn-1-yl)-3-(1-fluoro-2-(2-methyloxazol-4-yl)ethenyl)-8,8,12,16-tetramethyl-4,17-dioxabicyclo [14.1.0] heptadecane-5,9-dione;
(4S, 7R, 8S, 9S, 13E/Z, 16S (E))-4,8-dihydroxy-16-(1-chloro-2-(2-methylthiazol-4-yl)ethenyl)-1-oxa-5,5,9,13-tetramethyl-7-(prop-2-en-l-yl)-cydohexadec-13-ene-2,6-dione;
(4S, 7R, 8S, 9S, 13E/Z, 16S)-4,8-dihydroxy-16-(2-methyl-benzothiazol-5-yl)-1-oxa-5,5,9,13-tetramethyl-7-(prop-2-en-1-yl)-cyclohexadec-13-ene-2,6-dione;
(4S, 7R, 8S, 9S, 13E/Z, 16S (E))-4,8-dihydroxy-16-(1-methyl-2-(2-pyridyl)ethenyl)-1-oxa-5,5,9,13-tetramethyl-7-(but-3-yn-1-yl)-cyclohexadec-13-ene-2,6-dione;
(4S, 7R, 8S, 9S, 13E/Z, 16S (E))-4,8-dihydroxy-16-(1-methyl-2-(2-pyridyl)ethenyl)-1-oxa-5,5,9,13-tetramethyl-7-(but-3-en-1-yl)-cyclohexadec-13-ene-2,6-dione;
(1S, 3S (E), 7S, 10R, 11S, 12S, 16R)-7,11-dihydroxy-10-(prop-2-en-1-yl)-3-(I-methyl-2-(2-methylthlazol-4-yl)ethenyl)-8,8,12,16-tetramethyl-4,17-dioxabicyclo [14.1.0] heptadecane-5,9-dione;
(4S, 7R, 8S, 9S, 13E/Z, 16S (E))-4,8-dihydroxy-16-(1-methyl-2-(2-pyridyl)ethenyl)-I-oxa-5,5,9,13-tetramethyl-7-(prop-2-yn-1-yl)-cyclohexadec-13-ene-2,6-dione;
(4S, 7R, 8S, 9S, 13E/Z, 16S (E))-4,8-dihydroxy-16-(1-fluoro-2-(2-methylthiazol-4-yl)ethenyl)-1-oxa-5,5, 9, 13-tetramethyl-7-(prop-2-yn-l-yl)-cyclohexadec-13-ene- 2,6-dione;
(1S, 3S (E), 7S, 10R, 11S, 12S, 16R)-7,11-dihydroxy-10-(prop-2-yn-1-yl)-3-(1-fluoro-2-(2-methylthiazol-4-yl)ethenyl)-8,8,12,16-tetramethyl-4,17-dioxabicyclo [14.1.0] heptadecane-5,9-dione;
(4S, 7R, 8S, 9S, 13E/Z, 16S)-4,8-dihydroxy-16-(2-methyl-benzoxazol-5-yl)-l-oxa-5,5,9,13-tetramethyl-7-(prop-2-en-1-yl)-cyclohexadec-13-ene-2,6-dione;
(1R, 3S, 7S, 10R, 11S, 12S, 16S)-7,11-dihydroxy-10-(prop-2-en-1-yl)-3-(2-methyl-benzothiazol-5-yl)-8,8,12,16-tetramethyl-4,17-dioxabicyclo [14.1.0] heptadecane-5,9-dione;
(4S, 7R, 8S, 9S, 13E/Z, 16R)-4,8-dihydroxy-16-(2-methyl-benzothlazol-5-yl)-I-aza-5,5, 9,13-tetramethyl - 7- (prop-2-en -1-yl) -cyclohexadec-13-ene-2,6-dione;
(1S, 3S, 7S, 10R, 11S, 12S, 16R)-7,11-dihydroxy-10-(2-oxacyclopropyl-1-methyl)-3-(2-methyl-benzothlazol-5-yl)-8,8,12,16-tetramethyl-4,17-dioxablcyclo [14.1.0] heptadecane-5,9-dione;
(1R, 3S (E), 7S, 10R, 11S, 12S, 16S)-7,11-dihydroxy-10-(but-3-yn-1-yl)-3-(1-methyl-2-(2-pyridyl)ethenyl)-8,8,12,16-tetramethyl-4,17-dioxabicyclo [14.1.0] heptadecane-5,9-dione;
(4S, 7R, 8S, 9S, 13E/Z, 16S (E))-4,8-dihydroxy-16-(1-methyl-2-(2-methylthiazol-4-yl)ethenyl)-1-oxa-5,5,9,13-tetramethyl-7-(prop-2-en-1-yl)-cyclohexadec-13-ene-2,6-dione;
(1R, 3S (E), 7S, 10R, 11S, 12S, 16S)-7,11-dihydroxy-10-(prop-2-en-1-yl)-3-(l-methyl-2-(2-methylthlazol-4-yl)ethenyl)-8,8, 12, 16-tetramethyl-4, 17-dioxabicyclo [14.1.0] heptadecane-5,9-dione;
(4S, 7R, 8S, 9S, 13E/Z, 16S (E))-4,8-dihydroxy-16-(1-methyl-2-(-(2-pyridyl)ethenyl)-1-oxa-5,5,9,13-tetramethyl-7-(prop-2-en-1-yl)-cyclohexadec-13-ene-2,6-dione;
(4S, 7R, 8S, 9S, 13E/Z, 16S (E))-4,8-dihydroxy-16-(l-fluoro-2-(2-methyloxazol-4-yl)ethenyl)-1-oxa-5,5,9,13-tetramethyl-7-(prop-2-yn-1-yl)-cyclohexadec-13-ene-2,6-dione;
(4S, 7R, 8S, 9S, 13E/Z, 16S)-4,8-dihydroxy-16-(2-methyl-benzothiazol-5-yl)-1-oxa-5,5,9,13-tetramethyl-7-(3-methyl-but-2-en-1-yl)-cyclohexadec-13-ene-2,6-dione;
(1S, 3R, 7S, 10R, 11S, 12S, 16R)-7,11-dihydroxy-10-(prop-2-en-1-yl)-3-(2-methyl-benzothiazol-5-yl)-8,8,12,16-tetramethyl-4-aza-17-oxa-bicyclo [14.1.0] heptadecane-5,9-dione;
(1R, 3S, 7S, 10R, 11S, 12S, 16S)-7,11-dihydroxy-10-(prop-2-en-1-yl)-3-(quinolin-7-yl)-8,8,12,16-tetramethyl-4,17-dioxabicyclo [14.1.0] heptadecane-5,9-dione;
(1R, 3S, 7S, 10R, 11S, 12S, 16S)-7,11-dihydroxy-10-(prop-2-yn-1-yl)-3-(2-methyl-benzothiazol-5-yi)-8,8,12,16-tetramethyl-4,17-dioxabicyclo [14.1.0] heptadecane-5,9-dione;
(1S, 3S (E), 7S, 10R, 11S, 12S, 16R)-7,11-dihydroxy-10-(but-3-yn-1-yl)-3-(1-methyl-2-(2-pyridyl)ethenyl)-8,8,12,16-tetramethyl-4,17-dioxabicyclo [14.1.0] heptadecane-5,9-dione;
(1R, 3S (E), 7S, 10R, 11S, 12S, 16S)-7,11-dihydroxy-10-(but-3-yn-1-yl)-3-(l-methyl-2-(2-pyridyl)ethenyl)-8,8,12,16-tetramethyl-4,17-dioxabicyclo [14.1.0] heptadecane-5,9-dione;
(1S, 3S (E), 7S, 10R, 11S, 12S, 16R)-7,11-dihydroxy-10-(but-3-en-1-yl)-3-(1-methyl-2-(2-pyridyl)ethenyl)-8,8,12,16-tetramethyl-4,17-dioxabicyclo [14.1.0] heptadecane-5,9-dione:
(1R, 3S (E), 7S, 10R, 11S, 12S, 16S)-7,11-dihydroxy-10-(but-3-en-1-yl)-3-(l-methyl-2-(2-pyridyl)ethenyl)-8,8,12,16-tetramethyl-4,17-dioxabicyclo [14.1.0] heptadecane-5,9-dione;
(4S, 7S, 8R, 9S, 13E/Z, 16S (E))-4,8-dihydroxy-16-(1-methyl-2-(2-pyridyl)ethenyl)-1-oxa-5,5,9,13-tetramethyl-7-(but-3-yn-1-yl)-cyclohexadec-13-ene-2,6-dione;
(1'S, 4S, 7R, 8S, 9S, 13E/Z, 16S (E))-4,8-dihydroxy-7-(prop-2-en-1-yl)-16-(1'-methyl-2'-(pyridin-2-yl)ethyl)-5,5,9,13-tetramethyl-1-oxa-hexadec-13-ene-2,6-dione;
(1'S, 4S, 7R, 8S, 9S, 13E/Z, 16S (E))-4,8-dihydroxy-7-(prop-2-en-1-yl)-16-(1'-methyl-2'-(pyridin-2-yl)ethyl)-5,5,9,13-tetramethyl-1-oxa-hexadec-13-ene-2,6-dione;
(1S/R, 3S (E), 7S, 10R, 11S, 12S, 16R/S)-7,11-dihydroxy-10-(2-oxacyclopropyl-1-methyl)-3-(1-methyl-2-(2-pyridyl)ethenyl)-8,8,12,16-tetramethyl-4,17-dioxabicyclo [14.1.0] heptadecane-5,9-dione;
(1S/R, 3S (E), 7S, 10R, 11S, 12S, 16R/S)-7,11-dihydroxy-10-(2-oxacyclopropyl-1-methyl)-3-(1-methyl-2-(2-pyridyl)ethenyl)-8,8,12,16-tetramethyl-4,17-dioxabicyclo [14.1.0] heptadecane-5,9-dione;
(1S, 3S (E), 7S, 10R, 11S, 12S, 16R)-7,11-dihydroxy-10-(2-oxacyclopropyl-1-methyl)-3-(1-methyl-2-(2-pyridyl)ethenyl)-8,8,12,16-tetramethyl-4,17-dioxabicyclo [14.1.0] heptadecane-5,9-dione;
(1R, 3S (E), 7S, 10R, 11S, 12S, 16S)-7,11-dihydroxy-10-(prop-2-yn-1-yl)-3-(1-fluoro-2-(2-methylthiazol-4-yl)ethenyl)-8,8,12,16-tetramethyl-4,17-dioxabicyclo [14.1.0] heptadecane-5,9-dione;
(1R, 3S (E), 7S, 10R, 11S, 12S, 16S)-7,11-dihydroxy-10-(prop-2-yn-1-yl)-3-(1-fluoro-2-(2-methylthiazol-4-yl)ethenyl)-8,8,12,16-tetramethyl-4,17-dioxabicyclo [14.1.0] heptadecane-5,9-dione;
(1R, 3S, 7S, 10R, 11S, 12S, 16S)-7,11-dihydroxy-10-(prop-2-en-1-yl)-3-(2-methyl-benzoxazol-5-yl)-8,8,12, 16tetramethyl-4,17-dioxablcyclo [14.1.0] heptadecane- 5, 9-dione;
(1R, 3S (E), 7S, 10R, 11S, 12S, 16S)-7,11-dihydroxy-10-(prop-2-yn-1-yl)-3-(1-fluoro-2-(2-methyloxazol-4-yl)ethenyl)-8,8,12,16-tetramethyl-4, 17-dioxablcydo [14.1.0] heptadecane-5,9-dione;
(4S, 7R, 8S, 9S, 13E/Z, 16S)-4,8-dihydroxy-16-(2-methyl-benzothiazol-5-yl)-1-oxa-5,5,9,13-tetramethyl-7-(prop-2-en-1-yl)-cyclohexadec-13-ene-2,6-dione;
(1R, 3S, 7S, 10R, 11S, 12S, 16S)-7,11-dihydroxy-10-(prop-2-en-1-yl)-3-(2-methyl-benzothiazol-5-yl)-8,8,12,16-tetramethyl-4,17-dioxabicyclo [14.1.0] heptadecane-5,9-dione;
(4S, 7R, 8S, 9S, 13E/Z, 16S)-4,8-dihydroxy-16-(2-methyl-benzothiazol-5-yl)-1-aza-5,5,9,13-tetramethyl-7-(prop-2-en-1-yl)-cyclohexadec-13-ene-2,6-dione;
(4S, 7R, 8S, 9S, 13E/Z, 16S)-4,8-dihydroxy-16-(2-methyl-benzothiazol-5-yl)-1-aza-5,5,9,13-tetramethyl-7-(prop-2-en-1-yl)-cyclohexadec-13-ene-2,6-dione;
(4S, 7R, 8S, 9S, 13E/Z, 16R)-4,8-dihydroxy-16-(2-methyl-benzothiazol-5-yl)-1-aza-5,5,9,13-tetramethyl-7-(prop-2-en-1-yl)-cyclohexadec-13-ene-2,6-dione;
(1R, 3S, 7S, 10R, 11S, 12S, 16S)-7,11-dihydroxy-10-(prop-2-en-1-yl)-3-(2-methyl-benzothiazol-5-yl)-8,8,12,16-tetramethyl-4-aza-17-oxa-bicyclo [14.1.0] heptadecane-5,9-dione;
(1S, 3S, 7S,10R, 11S, 12S, 16R)-7,11-dihydroxy-3-(2-methyl-benzothiazol-5-yl)-10-(prop-2-en-I-yl)-8,8,12,16-tetramethyl-4,17-dioxabicylo[14,1,0]heptadecane-5,9-dlone;
(4S, 7R, 8S, 9S, 13E/Z, 16S)-4,8-dihydroxy-16-(quinolin-7-yl)-1-oxa-5,5,9,13-tetramethyl-7-(prop-2-en-1-yl)-cyclohexadec-13-ene-2,6-dione;
(1S, 3S, 7S, 10R, 11S, 12S, 16R)-7,11-dihydroxy-10-(prop-2-en-1-yl)-3-(2-methyl-benzothiazol-5-yl)-8,8,12,16-tetramethyl-4-aza-17-oxa-bicyclo [14.1.0] heptadecane-5,9-dione;
(1S, 3S, 7S, 10R, 11S, 12S, 16R)-7,11-dihydroxy-10-(prop-2-en-1-yl)-3-(2-methyl-benzothiazol-5-yl)-8,8,12,16-tetramethyl-4-aza-17-oxa-bicyclo [14.1.0] heptadecane-5,9-dione;
(4S, 7R, 8S, 9S, 13E/Z, 16R)-4,8-dihydroxy-16-(2-methyl-benzoxazol-5-yl)-1-aza-5,5,9,13-tetramethyl-7-(prop-2-en-1-yl)-cyclohexadec-13-ene-2,6-dione;
(4S, 7R, 8S, 9S, 13E/Z, 16R)-4,8-dihydroxy-16-(2-methyl-benzoxazol-5-yl)-1-aza-5,5,9,13-tetramethyl-7-(prop-2-en-1-yl)-cyclohexadec-13-ene-2,6-dione;
(4S, 7R, 8S, 9S, 13E/Z, 16S)-4,8-dihydroxy-16-(2-methyl-benzoxazol-5-yl)-1-aza-5,5,9, 13-tetramethyl-7 -( prop- 2-en-l-yl)-cyclohexadec-13-ene-2,6-dione;
(1R, 3R, 7S, 10R, 11S, 12S, 16S)-7,11-dihydroxy-10-(prop-2-en-1-yl)-3-(2-methyl-benzoxazol-5-yl)-8,8,12,16tetramethyl-4-aza-17-oxa-bicyclo [14.1.0] heptadecane-5,9-dione;
(1S, 3R, 7S, 10R, 11S, 12S, 16R)-7,11-dihydroxy-10-(prop-2-en-1-yl)-3-(2-methyl-benzoxazol-5-yl)-8,8,12,16tetramethyl-4-aza-17-oxa-bicyclo [14.1.0] heptadecane-5,9-dione;
(1R, 3R, 7S, 10R, 11S, 12S, 16S)-7,11-dihydroxy-10-(prop-2-en-1-yl)-3-(2-methyl-benzoxazol-S-yl)-8,8,12,16tetramethyl-4-aza-17-oxa-bicyclo [14.1.0] heptadecane-5,9-dione;
(1S, 3S (E), 7S, 10R, 11S, 12S, 16R)-7,11-dihydroxy-10-(prop-2-en-1-yl)-3-(1-chloro-2-(2-methylthiazol-4-yl)ethenyl)-8,8,12,16-tetramethyl-4,17-dioxabicyclo [14.1.0] heptadecane-5,9-dione;
(1R, 3S (E), 7S, 10R, 11S, 12S, 16S)-7,11-dihydroxy-10-(prop-2-en-1-yi)-3-(1-chloro-2-(2-methylthiazol-4-yl)ethenyl)-8,8,12,16-tetramethyl-4,17-dioxabicyclo [14.1.0] heptadecane-5,9-dione;
(1S, 3S (E), 7S, 10R, 11S, 12S, 16R) -7, 11 -dihydroxy-10-(2-oxacyclopropyl- 1 -methyl)-3-(1-chloro-2-(2-methyl-thiazol-4-yl)ethenyl)-8,8,12,16-tetramethyl-4,17-dioxabicydo [14.1.0] heptadecane-5,9-dione;
(1R, 3S (E), 7S, 10R, 11S, 12S, 16S)-7,11-dihydroxy-10-(prop-2-en-1-yl)-3-(1-fluoro-2-(2-methyl-thiazol-4-yl)ethenyl)-16-hydroxymethyl-8,8,12-trimethyl-4,17-dioxabicyclo [14.1.0] heptadecane-5,9-dione;
(1S, 3S, 7S, 10R, 11S, 12S, 16R)-7,11-dihydroxy-10-(2-oxacyclopropyl-1-methyl)-3-(2-methyl-benzothiazol-5-yl)-8,8,12,16-tetramethyl-4,17-dioxabicyclo [14.1.0] heptadecane-5,9-dione;
(4S, 7R, 8S, 9S, 13E/Z, 16S (E))-4,8-dihydroxy-7-(prop-2-en-1-yl)-16-(1-fluoro-2-(2-methyl-thiazol-4-yl)ethenyl)-13-hydroxymethyl-5,5,9-trimethyl-1-oxa-hexadec-13-ene-2,6-dione;
(1S, 3S (E), 7S, 10R, 11S, 12S, 16R)-7,11-dihydroxy-10-(prop-2-en-1-yl)-3-(1-fluoro-2-(2-methyl-thiazol-4-yl)ethenyl)-16-hydroxymethyl-8,8,12-trimethyl-4,17-dioxabicyclo [14.1.0] heptadecane-5,9-dione;
(4S, 7S, 8R, 9S, 13E/Z, 16S)-4,8-dihydroxy-16-(2-methyl-benzothiazol-5-yl)-1-oxa-5,5,9, 13-tetramethyl-7 -(prop-2-en-l-yl)-cyclohexadec-13-ene-2,6-dione;
(1S, 3S, 7S, 10S, 11R, 12S, 16R)-7,11-dihydroxy-10-(prop-2-en-1-yl)-3-(2-methyl-benzothiazol-5-yl)-8,8,12,16-tetramethyl-4,17-dioxabicyclo [14.1.0] heptadecane-5,9-dione;
(1R, 3S, 7S, 10S, 11R, 12S, 16S)-7,11-dihydroxy-10-(prop-2-en-1-yl)-3-(2-methyl-benzothiazol-5-yl)-8,8,12,16-tetramethyl-4,17-dioxabicyclo [14.1.0] heptadecane-5,9-dione;
(4R, 7S, 8R, 9R, 13E/Z, 16R)-4,8-dihydroxy-16-(2-methyl-benzothiazol-5-yl)-1-oxa-5,5,9,13-tetramethyl-7-(prop- 2-en-1-yl)-cyclohexadec-13-ene-2,6-dione;
(1R, 3R, 7R, 10S, 11R, 12R, 16S)-7,11-dihydroxy-10-(prop-2-en-1-yl)-3-(2-methyl-benzothiazol-5-yl)-8,8,12,16-tetramethyl-4,17-dioxabicyclo [14.1.0] heptadecane-5,9-dione;
(1S, 3R, 7R, 10S, 11R, 12R, 16R)-7,11-dihydroxy-10-(prop-2-en-1-yl)-3-(2-methyl-benzothiazol-5-yl)-8,8,12,16-tetramethyl-4,17-dioxabicyclo [14.1.0] heptadecane-5,9-dione;
(1S, 3S (E), 7S, 10R, 11S, 12S, 16R)-7,11-dihydroxy-10-(but-3-yn-1-yl)-3-(1-methyl-2-(2-pyridyl)ethenyl)-8,8,12,16-tetramethyl-4,17-dioxabicyclo [14.1.0] heptadecane-5,9-dione;
(1S, 3S (E), 7S, 10R, 11S, 12S, 16R)-7,11-dihydroxy-10-(but-3-en-1-yl)-3-(1-methyl-2-(2-pyridyl)ethenyl)-8,8,12,16-tetramethyl-4,17-dioxabicyclo [14.1.0] heptadecane-5,9-dione;
(1S, 3S (E), 7S, 10R, 11S, 12S, 16R)-7,11-dihydroxy-10-(prop-2-en-1-yl)-3-(1-methyl-2-(2-methylthiazol-4-yl)ethenyl)-8,8,12,16-tetramethyl-4,17-dioxabicyclo [14.1.0] heptadecane-5,9-dione;
(1S, 3S, 7S, 10R, 115, 12S, 16 R)-7,11-dihydroxy- 10- (prop- 2-en-1-yl)-3-(2-methyl-benzoxazol-5-yl)-8,8,12,16-tetramethyl-4,17-dioxabicyclo [14.1.0] heptadecane-5,9-dione;
(1S, 3S (E), 7S, 10R, 11S, 12S, 16R)-7,11-dihydroxy-10-(prop-2-en-1-yl)-3-(1-chloro-2-(2-methylthiazol-4-yl)ethenyl)-8,8,12,16-tetramethyl-4,17-dioxabicyclo [14.1.0] heptadecane-5,9-dione;
(1S, 3S, 7S, 10R, 11S, 12S, 16R)-7,11-dlhydroxy-10-(prop-2-en-1-yi)-3-(2-methyl-benzothlazol-5-yl)- 8,8,1.2,16-tetramethyl-4,17-dioxabicyclo [14.1.0] heptadecane-5,9-dione;
(1S, 3S, 7S, 10R, 11S, 12S, 16R)-7,11-dihydroxy-10-(prop-2-en-1-yl)-3-(2-methyl-benzothiazol-5-yl)-8,8,12,16-tetramethyl-4,17-dloxabicyclo [14.1.0] heptadecane-5,9-dione;
(1S, 3S, 7S, 10R, 11S, 12S, 16R)-7,11-dihydroxy-10-(prop-2-en-1-yl)-3-(quinolin-7-yl)-8,8,12,16-tetramethyl-4,17-dioxabicyclo [14.1.0] heptadecane-5,9-dione;
(1S, 3S, 7S, 10R, 11S, 12S, 16R)-7,11-dihydroxy-10-(prop-2-en-1-yl)-3-(quinolin-7-yl)-8,8,12,16-tetramethyl-4,17-dioxabicyclo [14.1.0] heptadecane-5,9-dione;
(1R, 3S (E), 7S, 10R, 11S, 12S, 16S)-7,11-dihydroxy-10-(prop-2-yn-1-yl)-3-(1-chloro-2-(2-methylthiazol-4-yl)ethenyl)-8,8,12,16-tetramethyl-4,17-dioxabicyclo [14.1.0] heptadecane-5,9-dione;
(4S, 7R, 8S, 9S, 13E/Z, 16S)-4,8-dihydroxy- 16-(2-methyl-benzothiazol-5-yl)-1-oxa-5,5,9,13-tetramethyl-7-(prop-2-yn-1-yl)-cyclohexadec-13-ene-2,6-dlone;
(1S, 3S, 7S, 10R, 11S, 12S, 16R)-7,11-dihydroxy-10-(prop-2-yn-1-yl)-3-(2-methyl-benzothiazol-5-yl)-8,8,12,16-tetramethyl-4,17-dioxabicyclo [14.1.0] heptadecane-5,9-dione; and/or
(4S, 7R, 8S, 9S, 13E/Z, 16S)-4,8-dihydroxy-16-(quinolin-7-yl)-1-oxa-5,5,9,13-tetramethyl-7-(prop-2-en-1-yl)-cyclohexadec-13-ene-2,6-dione.

In an interesting embodiment, the Epothilone derivative is (IS, 3S, 7S,10R, 11S, 12S, 16R)-7,11-dihydroxy-3-(2-methyl-benzothiazol-5-yl)-10-(prop-2-en-1-yl)-8,8,12,16-tetramethyl-4,17-dioxabicyclo[14.1.0]heptadecane-5,9-dione.

The Epothilone may be used In any amount in the compositions. It is of course desirable to apply high concentrations if possible, such as Epothilone in an amount of at least 0.05% by weight in the solid compositions, such as the lyophilisate. It is considered that a maximal content of Epothilone may be in the order of 2, 3, 4, 5 or 10% by weight dependent on the amount and type of reconstitution liquid. Typically, the amount of Epothilone is in a range between 0.05% and 10%, preferably 0.1% and 4% by weight, more preferably about 0.2 and 2% by weight in the lyophilisate. With respect to the reconstituted solution, the concentration ranges between 0.2 mg/ml and 10 mg/ml, preferably between 0.5 mg/ml and 5 mg/ml, such as about 1 mg/ml.

It is envisaged that the large size of the Epothilone of the invention compromises the formation of an inclusion complex wherein the Epothilone completely fits into the cavity of the cyclodextrin.

The term "cyclodextrin" is meant to define compounds comprising glucose units combined in a circular structure, namely compounds comprising 7 anhydro glucose units (β-cyclodextrin); 8 anhydro glucose units (γ-cyclodextrin) or 6 anhydro glucose units (α-cyclodextrin) as well as derivatives thereof. Each of the glucose units contains in 2-, 3-, and 6-position three hydroxy groups, which may be etherified or esterified, preferably etherified. Thus, the term " cyclodextrin derivative" encompasses herein etherified and esterified cyclodextrins. It is to be understood that the cyclodextrin may be completely or partially etherified or esterified, which means that all or only a part of the hydroxy groups are derivatised to form an ether or ester. Thus, at least 10%, such as least 20, 30, 40, 50, 60, 70, 80 or 90% of the alcohol groups may be alkylated or acylated. It should also be understood that no more than 40%, 30%, 20%, 10% or 5% of the 5% of the alcohol groups may be alkylated or acylated.

In preferred embodiments of the Invention the cyclodextrin is β-cyclodextrin or a derivative thereof, such as an alkylated cyclodextrin, I.e. alkyl ether cyclodextrin. The alkyl may be of any carbon length, though preferably less than 10 carbon atoms, preferably less than 5 carbon atoms, such as an alkyl selected from methyl, ethyl, propyl, butyl or pentyl including branched chains thereof (iso-propyl).

In still preferred embodiments of the invention, the alkyl of the alkylated cyclodextrin contains a functional group such as hydroxy group and/or sulphur group. Thus, in preferred embodiments of the Invention, the cyclodextrin derivative, such as a β-cyclodextrin derivative is etherified with hydroxyalkyl groups and/or sulfoalkyl groups resulting in hydroxyalkylated cyclodextrins (e. g.hydroxymethylated, hydroxyethylated, hydroxypropylated, hydroxybutylated, hydroxypentylated cyclodextrins), or sulfoalkylated cyclodextrins (e. g., sulfomethylated, sulfoethylated, sulfopropylated, sulfobutylated, sulfopentylated cyclodextrins).

The etherification of a cyclodextrin with alkyl groups may be stated directly as degree of substitution (DS) per glucose unit, which as stated above is 3 for complete substitution. Partially etherified cyclodextrins are used within the invention, which comprise alkyl groups, such as hydroxyalkyl groups and sulfoalkyl groups as described above, up to a degree of substitution of 0.05 to 2.0, preferably 0.2 to 1.5. Most preferably the degree of substitution with alkyl groups is between about 0.5 and about 1.2 per glucose unit.

Some embodiments of the Invention relates to compositions containing Epothilone and cyclodextrin in a specific molar ratio of Epothilone to cyclodextrin of 1:8 to 1:100, preferred 1:11 to 1:80 which is equivalent to the mass ratio of 1:21 to 1:300, preferred 1:29 to 1:200. Especially preferred are the ratios obtained by the examples herein.

The cyclodextrin may be used in any amount in the compositions depending on the type of cyclodextrin. Preferably, the amount ranges between 10 and 99.8 % by weight in the solid compositions, such as in the lyophilisate. More preferably, the amount ranges between 30 and 98 %, most preferably between 50 and 98%, such as 69 and 96% by weight. With respect to the reconstituted solution, the concentration ranges between 10 mg/ml and 1000 mg/ml, preferably 50 mg/ml and 500 mg/ml, such as about 200 mg/ml.

In a preferred embodiment of the invention, the Epothilone is an Epothilone derivative according to formula I above and the cyclodextrin is a sulfoalkylated β-cyciodextrin, such as sulfomethylated, sulfopenthylated, sulfopropylated, sulfobutylated, sulfopentylated β-cyclodextrin, preferably sulfobutylated β-cyclodextrin. In other words, the cyclodextrin is sulfomethylether-β-cyclodextrin, sulfoethylether-β-cyclodextrin, sulfopropylether-β-cyclodextrin, sulfobutylether-β-cyclodextrin, sulfopentylether-β-cyclodextrin, preferably sulfobutylether-β-cyclodextrin.

In another preferred embodiment of the invention, the Epothilone is an Epothilone derivative according to formula I above and the cyclodextrin is a hydroxyalkylated β-cyclodextrin, such as hydroxymethylated, hydroxyethylated, hydroxypropylated, hydroxybutylated, hydroxypentylated β-cyclodextrin, preferably hydroxypropylated p-cyclodextrin, provided that said Epothilone and cyclodextrin Is further combined with at least one pharmaceutically acceptable agent selected from a tonicifying agent, a filler and a buffering agent. In other words, the cyclodextrin may be hydroxymethylether-l3-cyclodextrin, hydroxyethylether-β-cyclodextrin, hydroxypropylether-β-cyclodextrin, hydroxybutylether-β-cyclodextrin, hydroxypentylether-β-cyclodextrin, preferably hydroxypropylether-β-cyclodextrin.

A still further aspect of the invention relates to a composition comprising an Epothilone according to formula I above and a β-cyclodextrin, preferably a hydroxyalkylated β-cyclodextrin or a sulfoalkylated β-cyclodextrin mentioned above, the composition may be an inclusion complex or whatever else these two components may have built up.

Commercially available cyclodextrins of interest include alkyl and allyl derivatives, hydroxyalkyl derivatives such as gamma-cyclodextrin, hydroxypropyl-gamma-cyclodextrin, hydroxypropyl-beta-cyclodextrin, sulfobutylether-beta-cyclodextrin, methyl-beta-cyclodextrin, methylthioureido-beta-cyclodextrin, propanediamine-beta-cyclodextrin, ethanedlamine-beta-cyclodextrin, hydroxyethylamino-beta-cyclodextrin.

As said compositions may further comprise at least one pharmaceutically acceptable agent selected from a tonicifying agent, a filler or a buffering agent.

The terms "tonicifying agent" and "tonicity modified" are interchangeable terms referring to a compound that upon dissolving in the composition at the time of use, provides a tonicity within the physiological range of the blood, peritoneal fluid or other relevant body fluids. Obviously, the addition of a "tonicifying agent may also depend on whether the solvent for reconstitution comprises tonicity-modifying agents. The resulting solution, e.g. the reconstituted solution, may have an osmolality in the range of about 100 to 900 mOsm/kg H₂O- Preferably, the osmolality is in the range of about 150 to 900mOsm/kg H₂O, more preferably in the range of about 200 to 500 mOsm/kg H₂O, Still more preferably in the range of about 250 to 350 mOsm/kg H₂O, Most suitable, the osmolality Is in the range of about 280 to 320 mOsm/kg H₂O.

As used herein a tonicifying agent may be a polyol, such as mannitol, sorbitol and xylitol , preferably mannitol or sodiumchloride.

In some embodiments, the addition of a tonicity modifying agent is not required because the composition has the desired osmolality. The inventors have found that a suitable tonicity is achieved when used as the cyclodextrin, a sulfoalkyl ether β-cyclodextrin. Thus, in embodiments applying a sulfoalkyl ether β-cyclodextrin as the cyclodextrin, any tonicity modifying agent Is not required or the sulfoalkyl ether β-cyclodextrin may be used in an amount not affecting the tonicity of the composition.

In a preferred embodiment of the Invention, at least one further pharmaceutically acceptable excipient, which need to be to be added to the combination of an Epothilone and a cyclodextrin is mannitol, sorbitol and/or xylitol. The addition of mannitol, sorbitol and/or xylitol may serve different functions. In general it is known that mannitol is a filler (bulking agent) suitable for lyophilised compositions, and as mentioned mannitol has also tonicity modifying activity when dissolved in a liquid composition, such as the composition resulting from reconstitution of the lyophilisate with a suitable solvent.

The term "cryoprotectants" as used herein generally Include agents, which provide stability to the Epothilone from freezing-induced stresses. Examples of cryoprotectants Include cyclodextrins such as β-cyclodextrin and derivatives thereof, and Include polyols such as, for example, mannitol, as well as pH regulators. The pH regulators include amines, such as trometamol, mineral acids, organic acids, such as hydrochloric acid. Additionally surfactants can be used as cryoprotectants. Examples of surfactants Include amines such as, trometamol. Cryoprotectants also contribute to the tonicity of the formulations. Cryoprotectants may also have lyoprotectant effects.

The term "lyoprotectant" as used herein Includes agents that provide stability to the Epothilone during water removal in the drying process, such as during lyophilisation process. Examples of lyoprotectants include saccharides, in particular sugar alcohols In particular mannitol. Saccharides of interest are di- and tri-saccharides such as sucrose, dextrose, lactose, maltose and/or trehalose.

The term "filler" or "bulking agent" is interchangeable terms denoted to mean an agent providing good lyophilised cake properties, which form a pharmaceutically elegant product, which help the Epothilone or a derivative thereof to overcome various stresses, shear/freezing for example, associated with lyophilization processes. Furthermore, a filler helps to maintain the therapeutically activity levels during the freeze-drying process and subsequent storage. Typical examples on bulking agents include cyclodextrins, sugar alcohols, such as mannitol. These agents may also contribute to the tonicity of the formulations.

It is envisaged that the required amount of tonicity modifier, bulking agent, lyoprotectant, or cryoprotectant may depend on several factors such as the desired osmolality, stability, lyophilisation process and reconstitution characteristics. With respect to mannitol, it has been found that the required amount ranges between 0.5 and 50 % by weight in the solid compositions, such as in the lyophilisate. More preferably, the amount ranges between 2 and 20 %. With respect to the reconstituted solution, the concentration ranges between 1 mg/ml and 200 mg/ml, preferably between 2 mg/ml and 100 mg/ml, more preferably between 5 mg/ml and 50 mg/ml, such as about 20 mg/ml.

As said the compositions of the invention may further comprise a pH regulator, such as a buffering compound. It is envisaged that a pH regulator may be applied In order to further stabilise the Epothilones that are easily hydrolysed in aqueous solution having pH above neutral.

The term "pH regulator" is meant to encompass compounds that are suitable for keeping/maintaining the pH in the range of 4 to 9 In the reconstituted solution. As used herein, the pH regulator preferably maintain the pH in the range of 5 to 8, more preferably in the range of 6 to 7.5. Therefore, in a still further interesting embodiment of the invention, the pH of the compositions is kept within the pH range of within of 5 to 8, more preferably in the range of 6 to 7.5. That is to say that the pH in the Epothilone solution at the time before removing the moisture content, e.g. before freeze-drying, should be kept within a pH of 5 to 8. Advantageously, this pH range is also within the desired physiological range, thereby causing no harm to the user upon administering the composition by parenteral means. Preferably the pH is about neutral, such as close to a pH of 7.4.

Typical examples of pH regulators are TRIS, the acid form or salts of citric acid, acetic acid, histidine, malic acid, phosphoric acid, tartaric acid, succinic acid, MES, HEPES, imidazole, lactic acid, glutaric acid and glycylglycine. In one embodiment TRIS is used alone and in another embodiment TRIS is used together with hydrochloric acid.

By the term "TRIS" is understood 2-Amino-2-hydroxymethyl-1,3-propandiol, which also are known under the names trometamol; trimethylol aminomethane; tris(hydroxymethyl)aminomethane; trismanine; tris buffer; tromethane; THAM; Talastrol; Tris Amino and Tromethamine.

The pH-regulator may be used In any amount. Though, it has been found that the required amount ranges between 0.05 and 4.2 % in the solid compositions, such as in the lyophilisate. More preferably, the amount ranges between 0.2 and 1.5% by weight. With respect to the reconstituted solution, the concentration ranges between 0.1 mg/ml and 10 mg/ml, preferably between 0.2 mg/ml and 5 mg/ml, more preferably between 0.5 mg/ml and 3 mg/ml, such as about 1.2 mg/ml. Furthermore, the pH regulator may be used together with an acid or base, such as hydrochloric acid. The acid may be used in any amount. Though, it has been found that the required amount ranges between 0.01 and 0.9 % in the solid compositions, such as in the lyophilisate. More preferably, the amount ranges between 0.05 and 0.3 %. With respect to the reconstituted solution, the concentration ranges between 0.03 mg/ml and 2 mg/ml, preferably between 0.05 mg/ml and 1 mg/ml, more preferably between 0.1 mg/ml and 0.6 mg/ml, such as about 0.3 mg/ml.

It should be understood that the compositions of the invention are made without adding a surfactant. Thus, in interesting embodiments of the invention, the composition excludes a surfactant or at least is substantially free of surfactant. The term "surfactant" generally includes an agent, which protect the Epothilone or a derivative thereof from air/solution interface-induced stresses and solution/surface induced-stresses. For example, a surfactant may protect the Epothilone or a derivative thereof from aggregation. Suitable surfactants may Include certain amines, polysorbate or poloxamer such as Tween 20, Tween 80, or poloxamer 188.

It should be understood that the embodiments described herein may be combined in any suitable manner. Preferred embodiment of the invention Includes a composition comprising an Epothilone as defined herein, a cyclodextrin as defined herein and at least one pharmaceutically acceptable excipient selected from the group consisting of mannitol; sorbitol; xylitol; 2-Amino-2-hydroxymethyl-1,3-propandiol; the acid form or salts of citric acid, acetic acid, histidine, malic acid, phosphoric acid, tartaric acid, succinic acid, MES, HEPES, imidazole, lactic acid, glutaric acid and glycylglycine, preferably mannitol and/or TRIS.

Still preferably embodiments of the Inventions are compositions comprising;
i) Epothilone derivative in an amount of 0.1 - 2% by weight, preferably 0.2 - 1% by weight,
ii) hydroxyalkyl- β-cyclodextrin, preferably 2-Hydroxypropyl-β-cyclodextrin in an amount of 50 - 99% by weight, preferably 70 - 95% by weight,
iii) mannitol, xylitol or sorbitol, preferably mannitol In an amount of 0 - 50% by weight, preferably 1 - 20% by weight, more preferably 2-15% by weight,
iv) pH regulator, preferably Trometamol in an amount of 0 - 2% by weight, preferably 0.1 - 1% by weight,
v) Hydrochloric acid in an amount of 0 - 1% by weight, preferably 0.05 - 0.5% by weight.

Still preferably embodiments of the Inventions are compositions comprising;
i) Epothilone derivative in an amount of 0.01 - 2% by weight, preferably 0.02 - 1% by weight,
ii) sulfoalkyl- β-cyclodextrin, preferably sulfobutyl-β-cyclodextrin In an amount of 50 - 99.9% by weight, preferably 85 - 99.5% by weight,
iii) pH regulator, preferably Trometamol In an amount of 0 - 2% by weight, preferably 0.1 - 1% by weight, and
iv) Hydrochloric acid in an amount of 0 - 1% by weight, preferably 0.05 - 0.5% by weight.

Provided by the present invention are compositions with good chemical stability with respect to the Epothilone despite the fact that Epothilones are easily hydrolysed. The term "good chemical stability" Is meant to describe that the hydrolysis or otherwise chemical degradation of the Epothilone is minimised during storage or production of the compositions so that substantial preservation of the Epothilone is maintained.

Like-wise the chemical stability of the solid composition, such as the lyophilisate, is high. The stability is typically determined by storing the lyophilisate in a glass vial stopped with rubber stoppers and the amount of degradation product formed over a period of up to 1, 3, 6, or 9 months is analyzed by determining the formation of degradation products in each of the reconstituted solutions as a function of time and temperature. The concentration of Epothilone and its degradation products is determined using quantitative assays, such as by HPLC.

Epothilone is usually not very stable in solution. Thus the compound must be protected from hydrolysis. In addition the stability is dependent from the pH of the solution. The solubility under saturation conditions in water is about 12mg/l. The low stability of epothilone is shown in example 4. The degradation Is proportional to [K x concentration].

Example 6 refers to the stability of the lyophilisate and the figures 1 and 2 according to the invention illustrate the results:
Figure 1
   Assay of epothilone lyophilisate at various storage conditions
   The lyophilisate according to example 1 is stored at 6°C and 25°C respectively. It Is shown that the lyophilisate is stable during 18 month having a constant content of epothilone.
Figure 2
   The sum of impurities at various storage conditions
   The sum of impurities as they might increase if the compound were not instable is shown for the storage conditions of 6°C and 25°C. For both temperatures the impurities remained less than 1% and were constant during the whole range of time.

For example the stability of lyophilisate is such that less than 15% of the initial amount of Epothilone in the composition is degraded over a period up to 3 months when the composition is stored as sealed in the dark at 2°C to 8°C. Preferably less than 10%, such as less than 5% of the Initial amount of Epothilone in the composition is degraded at the stated conditions.

The term "initial content" relates to the amount of Epothilone added to a composition at the time of preparation. The concentration given herein (mg/ml) refers to either the concentration in the solution of Epothilone before removing the moisture (e.g. before freeze-drying) or Is referred as % w/w, which then relates to the concentration in the solid composition, e.g. the lyophilised cake.

A still further aspect of the invention relates to the manufacturing of a solid composition, such as a lyophilisate, according to the invention, which may be formed from solutions (hereinafter referred to as "original solutions") comprising an Epothilone described herein, a cyclodextrin described herein and optionally at least one further pharmaceutically acceptable excipient as defined herein above.

In one embodiment the method for producing a composition of the invention comprises the steps of
a) solving an Epothilone as defined herein in an organic solvent, such as an alcohol (preferably ethanol); and
b) solving a cyclodextrin as defined herein in aqueous solution, optionally together with at least one further pharmaceutical acceptable ingredient as defined herein, such as mannitol and /or tromethamol; optionally
c) adjusting the pH of the resulting mixture of b) to a pH ranging between 5 and 9, preferably 6 and 8, such as about 7.4 using an inorganic acid, such as hydrochloric acid; and
d) mixing the resulting solvents a) and b) or a) and c); and optionally
e) carrying out sterile filtering of d) to achieve the so-called "original solution"
f) drying the solution so as to remove the solvent resulting in a solid composition.

In another embodiment, the method for producing a composition of the invention comprises the steps of
a) solving an Epothilone as defined herein in an organic solvent, such as an alcohol (preferably ethanol); and
b) evaporating said organic solvent; and
c) solving a cyclodextrin as defined herein in aqueous solution, optionally together with at least one further pharmaceutically acceptable ingredient as defined herein, such as mannitol and /or tromethamol; optionally
d) adjusting the pH of the resulting mixture of b) to a pH ranging between 5 and 9, preferably 6 and 8, such as about 7.4 using an inorganic acid, such as hydrochloric acid; and
e) solving the resulting powder b) in the resulting solvents c) or d); and optionally
f) carrying out sterile filtering of e) to achieve the so-called "original solution"
g) removing the solvent from the "original solution" to provide a solid composition.

Suitably, the drying process Is provided by lyophilisation or rotation evaporator.

In one aspect of the invention for the above methods Hydroxypropyl-β-cyclodexttrin Is used.

In another aspect of the invention for the above methods sulfobutylether-β-cyclodextrin is used.

The lyophilisate according to the second process mentioned above contains a higher amount of epothilone in the composition if amorphous epothilone is used for at least step e). It seems to be of no importance by which procedure the amorphous epothilone was obtained. The extend to which the amount of epothilone could be enhanced in comparison with the processes known of the art was surprising and not expected. Another aspect of the Invention refers to the fact that the amount of the cyclodextrin for the composition to be used for intravenous administration could be significantly reduced in comparison to the compositions known from the art e.g. as shown in example 1.

The compositions of the invention may be used for the treatment of a disease or condition associated with cell growth, division and/or proliferation, such as for treating malignant tumors in an Individual in need thereof. As applications, there can be mentioned, for example, the therapy of ovarian, stomach, colon, adeno-, breast, lung, head and neck carcinomas, malignant melanoma, acute lymphocytic, myelocytic leukaemia, bone-metastasis, and brain tumours. The compositions according to the invention are also suitable for treatment of chronic inflammatory diseases, such as, for example, psoriasis or arthritis. It follows that the compositions may be used for the preparation of a medicament for the treatment of the above-mentioned diseases.

Another further aspect of the Invention relates to methods of treating diseases and conditions associated with cell growth, division and/or proliferation in patients comprising administering to the patient a therapeutically effective of one or more compound of formula I using the compositions of the present invention, wherein said compositions are administered by intravenous infusion over a period of about 30 minutes in a dose ranging from 10 mg/m² to 35 mg/m², preferably from 16 mg/m² to 29 mg/m², most preferably 22 mg/m². The methods of the present invention also encompass dosing schedules, such as administration of the compositions of the invention to the patient once every 3 weeks or weekly for 3 weeks followed by one week of recovery or rest. The compositions are administered until progression or until the occurrence of unacceptable toxicities (i.e. Dose-Limiting Toxicities). A further aspect of the invention provides compositions that upon parenteral administration, such as by intravenous injection, result in a high Maximum Tolerated Dose (MTD), such as above 10 mg/m², preferably above 16 mg/m², more preferably above 22 mg/m². The MTD of an Epothilone administered in the form of a reconstituted composition of the present invention, may be observed in standard animal tests and in clinical trials.

### EXAMPLES

Although the examples are shown to having used a specific Epothilone derivative, named as Epothilone*, the invention should not be regarded as to be limited to this Epothilone derivative as it can be extended to many other Epothilone derivatives as defined above.

### Example 1

Composition comprising hydroxypropyl-β-cyclodextrin and the Epothilone derivative*: 1S, 3S, 7S, 10R, 11S, 12S, 16R)-7,11-dihydroxy-3-(2-methyl-benzothiazol-S-yl)-10-(prop-2-en-1-yl)-8,8,12,16-tetramethyl-4,17-dloxablcyclo[14.1.0]heptadecane-5,9-dione.

| **Ingredients** | **Lyophilisate (mg)** | **Lyophilisate (%)** | **Reconstituted composition (mg/ml)** |
|---|---|---|---|
| Epothilone derivative* | 10.500 | 0.449 | 1.000 |
| 2-Hydroxypropyl-β-cyclodextrin | 2100.000 | 89.879 | 200.000 |
| Mannitol | 210.000 | 8.988 | 20.000 |
| Trometamol | 12.705 | 0.544 | 1.210 |
| Hydrochloric acid | 3.267 | 0.140 | 0.311 |
| Total | 2336.472 | | |

For the method please see example 3.

The freeze-dried product (lyophilisate) is reconstituted by adding 8.8 ml of water for injection.

### Example 2

Composition comprising sulfobutyl-ether-β-cyclodextrin and the Epothilone derivative^{*}: 1S, 3S, 7S, 10R, 11S, 12S, 16R)-7,11-dihydroxy-3-(2-methyl-benzothiazol-5-yl)-10-(prop-2-en-1-yl)-8,8,12,16-tetramethyl-4,17-dioxabicyclo[14.1.0]heptadecane-5,9-dione.

| **Ingredients** | **Quantity In the lyophilised mass mg** | **Concentration after reconstitution mg/ml** |
|---|---|---|
| Epothilone * | 5.500 | 1.000 |
| Sulfobutyl-ether-β-cyclodextrin | 1,100.000 | 200.000 |
| Trometamol | 6.655 | 1.210 |
| Hydrochloric acid | ad pH 7.4 (in solution) | ad pH 7.4 |

In the first production step the Epothilone^{*} is dissolved in ethanol 96%. In the second production step sulfobutylether-β-cyclodextrin is dissolved in with water for injection. Trometamol is subsequently added to the cyclodextrin solution.

The resulting solution of sulfobutylether-β-cyclodextrin, is adjusted to pH 7.4 by adding diluted hydrochloric acid. Then the Epothilone solution and the cyclodextrin solution are combined and freeze dried under the following conditions: Freezing to -45°C at 1013 mmbar for up to 24 hours, preferably for 5 hours, primary drying step to 15°C at 8.9X10⁻² mmbar for 60 hours, preferably for 48hours, primary drying phase at 25°C at 8.9x10⁻² mmbar for 2 hours, preferably for 1 hour and secondary drying step at 25°C at 6.5 X10⁻³ mmbar for 10 hours, preferably for 6 hours using freeze dryer Fa. Hof, type COM0590.

### Example 2A

Composition comprising sulfobutyl-ether-β-cyclodextrin and the Epothilone derivative*: 1S, 3S, 7S, 10R, 11S, 12S, 16R)-7,11-dlhydroxy-3-(2-methyl-benzothlazol-5-yl)-10-(prop-2-en-1-yl)-8,8,12,16-tetramethyl-4,17-dioxabicyclo[14.1.0]heptadecane-5,9-dione.

| **Ingredients** | **Solution mg/ml** | **Molar ratio DS/CD (mol)** | **Mass ratio DS/CD (mg)** |
|---|---|---|---|
| Epothilone * | 3.0 | 1 | 1 |
| Sulfobutyl-ether-β-cyclodextrln | 100.000 | 8,39 | 100 |
| Water for injection | ad 1 ml | | |

In the first production step the Epothilone derivative* was dissolved in an organic solvent and the solvent was subsequently evaporated off. In the second production step sulfobutylether-β-cyclodextrin was dissolved in water for injection. In the third production step the Epothilone* powder obtained from the first production step was dissolved in the aqueous solution obtained by the second production step.

The total stirring time for that process was 2 days.

### Example 2B

Composition comprising sulfobutyl-ether-β-cyclodextrin and the Epothilone derivative*: 1S, 3S, 7S, 10R, 11S, 12S, 16R)-7,11-dlhydroxy-3-(2-methyl-benzothiazol-5-yl)-10-(prop-2-en-l-yl)-8,8,12,16-tetramethyl-4,17-dioxablcyclo[14.1.0]heptadecane-5,9-dione.

| **Ingredients** | **Solution mg/ml** | **Molar ratio DS/CD (mol)** | **Mass ratio DS/CD (mg)** |
|---|---|---|---|
| Epothilone * | 2.6 | 1 | 1 |
| Sulfobutyl-ether-β-cyclodextrin | 100.000 | 9.68 | 38.46 |
| Water for injection | ad 1 ml | | |

In the first production step the Epothilone derivative^{*} was dissolved in an organic solvent and the solvent was subsequently evaporated off. In the second production step sulfobutylether-β-cyclodextrin was dissolved in water for injection. In the third production step the Epothilone* powder obtained from the first production step was dissolved in the aqueous solution obtained by the second production step.

The total stirring time for that process was 3 hours.

### Example 2C

Composition comprising sulfobutylether-β-cyclodextrin and the Epothilone derivative*: 1S, 3S, 7S, 10R, 11S, 12S, 16R)-7,11-dihydroxy-3-(2-methyl-benzothiazol-5-yl)-10-(prop-2-en-1-yl)-8,8,12,16-tetramethyl-4,17-dioxabicyclo[14.1.0]heptadecane-5,9-dione.

| **Ingredients** | **Solution (mg/ml)** | **Molar Ratio DS/CD (mol)** | **Mass Ratio DS/CD (mg)** |
|---|---|---|---|
| Epothilone derivative* | 1.000 | 1 | 1 |
| Sulfobutylether-β-cyclodextrin | 100.000 | 25.17 | 100.00 |
| Trometamol | 1.210 | | |
| Hydrochloric acid | Ad pH 7.4 | | |
| Ethanol 96% | 12.15 | | |
| Water for Injection | Ad 1 ml | | |

In the first production step the Epothilone* was dissolved in ethanol 96%. In the second production step sulfobutylether-β-cyclodextrin was dissolved in water for injection. Trometamol is subsequently added to the cylodextrin solution.

The resulting solution of sulfobutylether-β-cydodextrin and trometamol was adjusted to pH 7.4 by adding diluted hydrochloric acid. Then the Epothilone solution and the cyclodextrin solution are combined. The total stirring time for that manufacturing process was 2 hours.

The organic solvent preferably used for solving the Epothilone derivative in 2A and 2B is methylenechloride or ethanol 96%.

### Example 3

### Manufacturing process for an "original solution" from where a lyophilisate is prepared

| **Ingredients** | **Quantity in g** |
|---|---|
| Epothilone* | 14.250 |
| Hydroxypropyl-β-cyclodextrin | 2850.000 |
| Mannitol | 285.000 |
| Trometamol | 17.243 |
| Hydrochloric acid | 44.336 |
| Ethanol 96% - processing aid | 173.850 |
| Water for lnjection - | 11862.822 |
| processing aid | |

| | |
|---|---|
| *(1S, 3S, 7S, 10R, 11S, 12S, 16R)-7,11-dihydroxy-3-(2-methyl-benzothiazol-5-yl)-10-(prop-2-en-1-yl)-8,8,12,16-tetramethyl-4,17-dioxabicyclo[14.1.0]heptadecane-5,9-dione. | |

In the first production step the Epothilone^{*} Is dissolved in ethanol 96%. In the second production step hydroxypropyl-β-cyclodextrin is dissolved in with water for injection. Trometamol is subsequent added to the cyclodextrin solution and then mannitol.

The resulting solution of hydroxypropyl-β-cyclodextrin, trometamol and mannitol is adjusted to pH 7.4 by adding diluted hydrochloric acid.

Then the Epothilone solution and the cyclodextrin solution are combined and freeze dried under the following conditions: Freezing to -45°C at 1013 mmbar for up to hours, preferably for 5 hours, first main drying phase to 15°C at 8.9X10⁻² mmbar for 60 hours, preferably for 48 hours, second main drying phase at 25°C at 8.9x10⁻² mmbar for 2 hours, preferably for 1 hour and postdrying phase at 25°C at 6.5 x10⁻³ mmbar for 10 hours, preferably for 6 hours using freeze dryer Fa. Hof, type COM 0590.

in order to obtain a solid composition as shown in Example 1.

### Example 4

Stability data of Epothilone* (1S, 3S, 7S, 10R, 11S, 12S, 16R)-7,11-dihydroxy-3-(2-methyl-benzothiazol-5-yl)-10-(prop-2-en-1-yl)-8,8,12,16-tetramethyl-4,17-dioxabicyclo[14.1.0]heptadecane-5,9-dione) in aqueous solutions containing hydroxypropyl-beta-cyclodextrin.

Freeze-dried preparations as shown in Example 1 were reconstituted with water for injection and the pH was then adjusted to pH values between 6.1 and 13.6 using HCI or NaOH. The solutions were left at room temperature or 40°C and assay and purity were measured at Intervals using HPLC.

The degradation rate constant (K) of the Epothilone^{*} decreases as the pH value approaches the neutral pH. The degradation rate is at minimum at pH 7.

| **pH** | **K at room temperature** | **K (40°)** |
|---|---|---|
| 9 | 4.5 10⁻³ h⁻¹ | |
| 8.0 | 1.1 10⁻³ h⁻¹ | 4.9 10⁻³ h⁻¹ |
| 7.5 | 0.7 10⁻³ h⁻¹ | |
| 6.9 | 0.1 10⁻³ h⁻¹ | 1.2 10⁻³ h⁻¹ |
| 6.3 | 0.5 10⁻³ h⁻¹ | 1.7 10⁻³ h⁻¹ |
| 6.1 | 0.8 10⁻³ h⁻¹ | |

### Example 5

Determination of the apparent equilibrium stability constant of the complex between Epothilone*(1S, 3S, 7S, 10R, 11S, 12S, 16R)-7,11-dihydroxy-3-(2-methylbenzothiazol-5-yl)-10-(prop-2-en-1-yl)-8,8,12,16-tetramethyl-4,17-dloxablcydo[14,1,0]heptadecane-5,9-dione) and hydroxypropyl-beta-cyciodextrin on aqueous solution.

The phase solubility diagram technique (PSD) was applied at 25°C and the stability constant of an assumed 1:1 complex are K'= 484.5M⁻¹. The solubility of the Epothilone* was Sₒ= 4.3 10⁻⁵ mol/l (0.023g/01). K' leads to the assumption that if at all a complex was formed it is not sufficiently stable.

### Example 6

Stability of the lyophilisate and of reconstituted lyophilisate containing Epothilone^{*} at various storage conditions:

### Stability of the Lyophilisate

See Figures 1 and 2

### Figure 1

### Assay of epothilone lyophilisate at various storage conditions

The lyophilisate according to example 1 is stored at 6°C and 25°C respectively. It is shown that the lyophilisate is stable during 18 month having a constant content of epothilone.

### Figures 2

### The sum of impurities at various storage conditions

The sum of impurities as they might increase if the compound were not instable is shown for the storage conditions of 6°C and 25°C. For both temperatures the impurities remained less than 1% and were constant during the whole range of time.

### Stabiliy of the Reconstituted Lyophilisate

| | **Content Epothilone* [mg/mL]** | **pH** | **Osmolality [mmol/kg]** | **Density [g/mL]** | **Color** |
|---|---|---|---|---|---|
| Start | 0.99 | 7.41 | 355 | 1.0745 | ≥ Y7 |
| | | | | | |
| filtered | 1.02 | | | | |
| 6 h filtered | 1.01 1.01 | 7.41 | 350 | 1.0746 | ≥Y7 |
| 24 h filtered | 1.01 1.00 | 7.42 | 351 | 1.0750 | ≥ Y7 |

The stability of the reconstituted lyophilisate is granted for at least one day. This period of time is at maximum necessary for the clinicians to prepare and administer the composition to the patient.

### Example 7

### Administration of the Epothilone* Using the Composition of example 1 of the Invention

Patients and Methods: Patients with histologically confirmed advanced solid tumors that were resistant or refractory to conventional antineoplastic treatment were eligible for the trial. They received treatment with Epothilone^{*} as 30 minutes intravenous infusion in 3 weeks intervals. Treatment was continued until progression or the occurrence of unacceptable toxicities. The starting dose was 0,6 mg/m². Doses were escalated using a modified Fibonacci design.

Results: 47 patients have been enrolled at 12 different dose levels up to 29 mg/m². Dose limiting toxicities observed were CTC Grade 3 peripheral neuropathy at 16 mg/m² and CTC Grade 4 ataxia at 29 mg/m². No other DLTs were observed. Hematologic toxicities of maximum CTC Grade 2 were infrequent. Most common side effect was peripheral sensory neuropathy, mostly of Grade 1-2. No Grade 3-4 non-hematological toxicities were reported, with the exception of the previously-mentioned DLTs. Results show anti-tumor activity (including objective responses) in patients with breast cancer, NSCLC, cholangiocarcinoma, uveal melanoma and head and neck cancer and that the Epothilone^{*} can be administered every 3 weeks at doses up to 29 mg/m² without severe toxicity.

### Example 8

Composition comprising hydroxypropyl-p-cyclodextrin and the Epothilone derivative^{*}: 1S, 3S, 7S, 10R, 11S, 12S, 16R)-7,11-dihydroxy-3-(2-methyl-benzothiazol-5-yl)-10-(prop-2-en-1-yl)-8,8,12,16-tetramethyl-4,17-dioxabicyclio[14.1.0]heptadecane-5,9-dione.

| **Ingredients** | **Solution mg/ml** | **Molar ratio DS/CD** | **Mass Ratio DS/CD** |
|---|---|---|---|
| Epothilone derivative* | 1 | 1 | 1 |
| 2-Hydroxypropyl-β-cyclodextrin | 200.000 | 77.3 | 200 |
| Water for injection | Ad 1ml | | |

In the first production step hydroxypropyl-β-cyclodextrin was dissolved in water for injection. In the second production step the crystalline Epothilone^{*} was added to said solution under stirring. The epothilon has not completely been dissolved after 5 hours of stirring time, and even not after 10 hours of stirring time. The target concentration of ≥ 1 mg/mL was only achieved after 20 hours of stirring time. The resulting solution of Epothilone^{*} and hydroxypropyl-β-cyclodextrin, was adjusted to pH 7.4 by adding diluted hydrochloric acid.

### Example 9

Composition comprising hydroxypropyl-β-cyclodextrin and the Epothilone derivative^{*}: 1S, 3S, 7S, 10R, 11S, 12S, 16R)-7,11-dihydroxy-3-(2-methyl-benzothlazol-5-yl)-10-(prop-2-en-1-yl)-8,8,12,16-tetramethyl-4,17-dioxabicyclo[14.1.0]heptadecane-5,9-dione.

| **Ingredients** | **Solution (mg/ml)** | **Solution (%)** | **Molar Ratio DS/CD (mol)** | **Mass Ratio DS/CD** (mg) |
|---|---|---|---|---|
| Epothilone derivative* | 1.000 | 0.093% | 1 | 1 |
| 2-Hydroxypropyl-β-cyclodextiin | 200.000 | 18.691% | 77.73 | 200 |
| Mannitol | 20.000 | 1.869% | | |
| Trometamol | 1.210 | 0.113% | | |
| Hydrochloric acid | 0.311 | 0.029% | | |
| Ethanol 96% | 12.200 | 1.140% | | |
| Water for injection | 835.279 | 78.063% | | |
| Total | 1070.000 | | | |

In the first production step the crystalline Epothilone^{*} was dissolved in ethanol 96%. In the second production step hydroxypropyl-β-cyclodextrin was dissolved in water for injection.

Trometamol is subsequently added to the cyclodextrin solution and then mannitol.

The resulting solution of hydroxypropyl-β-cyclodextrin, trometamol and mannitol was adjusted to pH 7.4 by adding diluted hydrochloric acid. Then the Epothilone solution and the cyclodextrin solution are combined. The total stirring time for that manufacturing process was 2 hours. Finally the solution was freeze dried in order to obtain a solid composition as shown in Example 1.

### Example 10

Composition comprising hydroxypropyl-β-cyclodextrin and the Epothilone derivative^{*}: 1S, 3S, 7S, 10R, 11S, 12S, 16R)-7,11-dihydroxy-3-(2-methyl-benzothiazol-5-yl)-10-(prop-2-en-1-yl)-8,8,12,16-tetramethyl-4,17-dioxabicyclo[14.1.0]heptadecane-5,9-dione.

| **Ingredients** | **Solution (mg/ml)** | **Solution* (%)** | **Molar Ratio DS/CD (mol)** | **Mass Ratio DS/CD (mg)** |
|---|---|---|---|---|
| Epothilone derivative* | 7.000 | 0.654% | 1 | 1 |
| 2-Hydroxypropyl-β-cyclodextrin | 200.000 | 18.672% | 11.10 | 28.57 |
| Mannitol | 20.000 | 1.867% | | |
| Trometamol | 1.210 | 0.113% | | |
| Hydrochloric acid | ad pH 7.4 | | | |
| Water for injection | ad 1 ml | | | |

| | | | | |
|---|---|---|---|---|
| *Density= 1.0711 g/ml | | | | |

In the first production step the Epothilone derivative* was dissolved in an organic solvent, such as methylenchloride, and the solvent was subsequently evaporated off. In the second production step hydroxypropyl-β-cyclodextrin was dissolved in water for injection. Trometamol is subsequently added to the cyclodextrin solution and then mannitol. In the third production step the Epothilone* powder obtained from the first production step was dissolved in the aqueous solution obtained by the second production step. The resulting solution of Epothilone*, hydroxypropyl-β-cyclodextrin, trometamol and mannitol was adjusted to pH 7.4 by adding diluted hydrochloric acid.

The total stirring time for that process was 2 hours.

### Example 11

Composition comprising hydroxypropyl-β-cyclodextrin and the Epothilone derivative*: 1S, 3S, 7S, 10R, 11S, 12S, 16R)-7,11-dihydroxy-3-(2-methyl-benzothiazol-5-yl)-10-(prop-2-en-1-yl)-8,8,12,16-tetramethyl-4,17-dioxabicyclo[14.1.0]heptadecane-5,9-dione.

| **Ingredients** | **Solution (mg/ml)** | **Solution* (%)** | **Molar Ratio DS/CD (mol)** | **Mass Ratio DS/CD (mg)** |
|---|---|---|---|---|
| Epothilone derivative* | 1.000 | 0.097% | 1 | 1 |
| 2-Hydroxypropyl-p-cyclodextrin | 50.00 | 4.854% | 19.43 | 50 |
| Mannitol | 43.000 | 4.175% | | |
| Trometamol | 1.210 | 0.117% | | |
| Hydrochloric acid | ad pH 7.4 | | | |
| Water for injection | ad 1 ml | | | |

| | | | | |
|---|---|---|---|---|
| *Density: 1.030 g/ml | | | | |

In the first production step the Epothilone derivative* was dissolved in an organic solvent and the solvent was subsequently evaporated off. In the second production step hydroxypropyl-β-cyclodextrin was dissolved in water for injection. Trometamol is subsequently added to the cyclodextrin solution and then mannitol. In the third production step the Epothilone* powder obtained from the first production step was dissolved in the aqueous solution. The resulting solution of Epothilone*, hydroxypropyl-β-cyclodextrin, trometamol and mannitol was adjusted to pH 7.4 by adding diluted hydrochloric acid. The total stirring time for that process was 2 hours.

## Claims

1. Method of producing a composition comprising the steps of
(a) solving (1S, 3S, 7S,10R, 11S, 12S, 16R)-7,11-dihydroxy-3-(2-methyl-benzothiazol-5-yl)-10-(prop-2-en-1-yl)-8,8,12,16-tetramethyl-4,17-dioxabicyclo[14,1,0]heptadecane-5,9-dione in an organic solvent, such as an alcohol
and
b) solving a sulfobutyl ether-β-cyclodextrin as defined herein in aqueous solution, optionally together with
at least one further pharmaceutical acceptable ingredient as defined herein, such as mannitol and /or tromethamol; optionally
c) adjusting the pH of the resulting mixture of b) to a pH ranging between 5 and 9,
preferably 6 and 8, such as about 7.4 using an inorganic acid, such as hydrochloric
acid; and
d) mixing the resulting solvents a) and b) or a) and c); and optionally
e) carrying out sterile filtering of d) to achieve the so-called "original solution"
f) drying the solution so as to remove the solvent resulting in a solid composition.

2. Method of producing a composition comprising the steps of
a) solving (1S, 3S, 7S, 10R, 11S, 12S, 16R)-7,11-dihydroxy-3-(2-methyl-benzothiazol-5-yl)-10-(prop-2-en-1-yl)-8,8,12,16-tetramethyl-4,17-dioxabicyclo[14,1,0]heptadecane-5,9-dione in an organic solvent, such as an alcohol
and
b) evaporating said organic solvent; and
c) solving sulfobutyl ether-β-cyclodextrin as defined herein in aqueous solution, optionally together
with at least one further pharmaceutically acceptable ingredient as defined herein,
such as mannitol and /or tromethamol; optionally
d) adjusting the pH of the resulting mixture of b) to a pH ranging between 5 and 9,
preferably 6 and 8, such as about 7.4 using an inorganic acid, such as hydrochloric
acid; and
e) dissolving the resulting powder b) in the resulting solvents c) or d); and optionally
f) carrying out sterile filtering of e) to achieve the so-called "original solution"
g) removing the solvent from the "original solution" to provide a solid composition.

3. A method according to claims 1 or 2 wherein the organic solvent used for step (a) is an alcohol, especially ethanol.

4. A method according to any of the preceeding claims wherein (1S, 3 S, 7S,10R, 11S, 12S, 16R)-7,11-dihydroxy-3-(2-methyl-benzothiazol-5-yl)-10-(prop-2-en-1-yl)-8,8,12,16-tetramethyl-4,17-dioxabicyclo[14,1,0]heptadecane-5,9-dione is in amorphous form.

5. Pharmaceutical composition obtainable by the method of claim 1 or claim 2.

6. A composition comprising (1S, 3S, 7S, 10R, 11S, 12S, 16R)-7,11-dihydroxy-3-(2-methyl-benzothiazol-5-yl)-10-(prop-2-en-1-yl)-8,8,12,16-tetramethyl-4,17-dioxabicyclo[14,1,0]heptadecane-5,9-dione, sulfobutyl ether-β-cyclodextrin and at least one pharmaceutically acceptable excipient selected from the group consisting of mannitol; sorbitol; xylitol; 2-Amino-2-hydroxymethyl-1,3-propandiol; the acid form or salts of citric acid, acetic acid, histidine, malic acid, phosphoric acid, tartaric acid, succinic acid, MES, HEPES, imidazole, lactic acid, glutaric acid and glycylglycine.

7. The composition comprising (1S, 3S, 7S, 10R, 11S, 12S, 16R)-7,11-dihydroxy-3-(2-methyl-benzothiazol-5-yl)-10-(prop-2-en-1-yl)-8,8,12,16-tetramethyl-4,17-dioxabicyclo[14,1,0]heptadecane-5,9-dione, sulfobutyl ether-β-cyclodextrin and further comprising optionally a tonicityfier selected from mannitol, sorbitol and xylitol and further comprising a pH regulator selected from 2-Amino-2-hydroxymethyl-1,3-propandiol, the acid form or salts of citric acid, acetic acid, histidine, malic acid, phosphoric acid, tartaric acid, succinic acid, MES, HEPES, imidazole, lactic acid, glutaric acid and glycylglycine..

8. The composition according to claim 7, wherein the pH regulator is 2-Amino-2-hydroxymethyl-1,3-propandiol.

9. . The composition according to any of the preceding claims 6-8, wherein the composition is in the form of a lyophilisate.

10. The composition according to any one of claims 6-9, wherein the composition results from the re-constitution of the lyophilisate.

11. The composition according to claim 10, wherein the composition further comprises a solvent selected from aqueous solutions comprising 75-100% of water by volume, preferably 85%-100% by volume, more preferably 90-100% by volume, most preferably 95-100% by volume.

12. A method for treating cancer in a patient comprising administering said patient a therapeutically effective of one or more compound of formula I using the compositions of the present invention, wherein said compositions are administered by intravenous infusion over a period of about 30 minutes in a dose ranging from 10 mg/m² to 35 mg/m².

13. A method according to claim 12, wherein the dose is from 16 mg/m² to 29 mg/m²_{.}

14. A method according to claim 13, wherein the dose is 22 mg/m².

15. A method according to anyone of claims 12-14, wherein the compositions are administered to the patient every 3 weeks or weekly for 3 weeks followed by one week recovery.
